(19) Europäisches Patentamt
European Patent Office
Office européen des brevets

(11) EP 3 533 784 B1

(12) **EUROPEAN PATENT SPECIFICATION**

(45) Date of publication and mention of the grant of the patent:
**06.10.2021 Bulletin 2021/40**

(21) Application number: **18161508.9**

(22) Date of filing: **13.03.2018**

(51) Int Cl.:
*C07C 255/50* (2006.01) *C07D 215/06* (2006.01)
*C07D 221/12* (2006.01) *C07D 221/18* (2006.01)
*C07D 333/50* (2006.01) *C07D 235/18* (2006.01)
*C07D 239/26* (2006.01) *C07D 251/24* (2006.01)
*C07D 471/04* (2006.01) *C07C 233/65* (2006.01)
*C07D 263/57* (2006.01) *C07D 277/66* (2006.01)
*C07D 213/06* (2006.01) *C07D 307/77* (2006.01)
*C07D 307/91* (2006.01) *C09K 11/06* (2006.01)
*H01L 51/00* (2006.01) *H05B 33/14* (2006.01)

(54) **SPIRO BENZOANTHRACENE-FLUORENE DERIVATIVES AND THEIR USE IN ORGANIC ELECTRONIC DEVICES, DISPLAYS AND LIGHTING DEVICES**

SPIRO BENZOANTHRACEN-FLUOREN DERIVATE UND DEREN VERWENDUNG IN ORGANISCHEN ELEKTRONISCHEN VORRICHTUNGEN, ANZEIGEN UND BELEUCHTUNGSVORRICHTUNGEN

DERIVES SPIRO BENZOANTHRACENE-FLUORENE ET LEUR UTILISATION DANS LES DISPOSITIFS ÉLECTRONIQUES ORGANIQUES, ÉCRANS ET DISPOSITIFS D'ÉCLAIRAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2018 EP 18159248**

(43) Date of publication of application:
**04.09.2019 Bulletin 2019/36**

(73) Proprietor: **Novaled GmbH**
**01099 Dresden (DE)**

(72) Inventors:
- **Marin, Lidia**
  **01099 Dresden (DE)**
- **Galan, Elena**
  **01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(56) References cited:
**WO-A1-2016/182270 WO-A1-2018/050584**
**CN-A- 104 592 978 KR-A- 20090 011 488**
**KR-A- 20170 131 742 US-A1- 2017 321 118**

- **KIM J-Y ET AL: "Orange phosphorescent organic light-emitting diodes using new spiro[benzoanthracene-fluorene]-type host materials", DYES AND PIGMENTS, vol. 94, no. 2, 15 January 2012 (2012-01-15), pages 304-313, XP028465516, ISSN: 0143-7208, DOI: 10.1016/J.DYEPIG.2012.01.009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 533 784 B1

## Description

The present invention relates to an organic electronic device, a compound which may be comprised in the organic electronic device, an organic semiconducting layer comprising said compound and display or lighting devices comprising the organic electronic device.

BACKGROUND ART

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer (HTL), an emission layer (EML), an electron transport layer (ETL), and a cathode, which are sequentially stacked on a substrate. In this regard, the HTL, the EML, and the ETL are thin films formed from organic and / or organometallic compounds.

When a voltage is applied to the anode and the cathode, holes injected from the anode electrode move to the EML, via the HTL, and electrons injected from the cathode electrode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency.

Triazine- and pyrimidine-based compounds are widely used in organic electronics applications, especially as electron transport materials. Derivatives of triazine and pyrimidine show often low values of glass transition temperature (Tg). Low Tg compounds are not preferred as materials in organic electronics because of the detrimental effect of the low Tg on the durability and performance of devices comprising such compounds. Bulky molecular fragments are required to provide amorphous character to the electron transport material and increase the Tg. However, bulky molecular fragments can significantly disturb the charge carrier mobility which deteriorates the overall device performance.

Kim J-Y et al., Dyes and Pigments, 2012, 94(2), pages 304-313 discloses orange phophorescent organic light-emitting diodes using spiro[benzoantracene-fluorene]-type host materials.

It is, therefore, an object of the present invention to provide novel organic electronic devices and compounds for use therein overcoming drawbacks of the prior art, in particular to provide novel compounds which are suitable to improve the performance of organic electronic devices, in particular cd/A efficiency of OLED devices, in particular when used in an electron transport layer which may further comprise an additive.

DESCRIPTION OF THE INVENTION

The above problems are solved in accordance with the subject-matter of the independent claims. Preferred embodiments result from the subclaims.

The above object is achieved by an organic electronic device comprising, between an anode and a cathode, at least one layer selected from an electron injection layer, an electron transport layer or an electron generation layer, the layer comprising at least one compound of the following Formula (I):

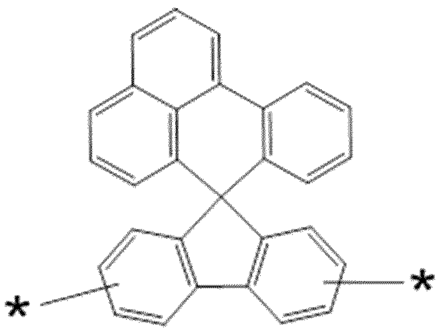

(I)

wherein

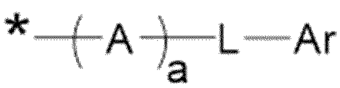

the compound of Formula (I) comprises one or more moieties $-(A)_a-L$ and the remaining positions marked with "*" are hydrogen or substituents independently selected from the group consisting of deuterium, fluorine, RF, $C_1$-$C_{20}$ linear alkyl, $C_3$-$C_{20}$ branched alkyl, $C_1$-$C_{12}$ linear fluorinated alkyl, CN, RCN, $C_6$-$C_{20}$ aryl, $C_2$-$C_{20}$ heteroaryl, $(P{=}O)R_2$; wherein each R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl;

A is selected from substituted or unsubstituted $C_6$-$C_{24}$ aryl or $C_2$-$C_{20}$ heteroaryl; wherein in case that A is substituted, the respective substituents are independently selected from the group consisting of deuterium, fluorine, $C_1$-$C_{20}$ linear alkyl, $C_3$-$C_{20}$ branched alkyl, linear fluorinated $C_1$-$C_{12}$ alkyl, CN, $C_6$-$C_{20}$ aryl, and $C_2$-$C_{20}$ heteroaryl;

L is selected from the group consisting of triazine, pyrimidine, benzoacridine, dibenzoacridine, pyridine, bi-pyridine, benzimidazole, phenanthroline, benzo-nitrile, phenanthridine, benzooxazole, benzothiazole, phenanthridine-one, naphto-benzofurane, di-naphtofurane, benzo-naphto-thiophene, dinaphtothiophene. or a polar group selected from

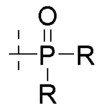 , 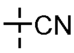 and 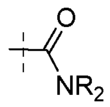,

wherein substituents, if present in the respective group L are independently selected from the group consisting of deuterium, fluorine, $C_1$-$C_{20}$ linear alkyl, $C_3$-$C_{20}$ branched alkyl, $C_3$-$C_{20}$ cyclic alkyl, $C_1$-$C_{20}$ linear alkoxy, $C_3$-$C_{20}$ branched alkoxy, $C_1$-$C_{12}$ linear fluorinated alkyl, $C_1$-$C_{12}$ linear fluorinated alkoxy, $C_3$-$C_{12}$ branched fluorinated cyclic alkyl, $C_3$-$C_{12}$ fluorinated cyclic alkyl, $C_3$-$C_{12}$ fluorinated cyclic alkoxy, CN, RCN, $C_6$-$C_{20}$ aryl, $C_2$-$C_{20}$ heteroaryl, OR, SR, $(C{=}O)R$, $(C{=}O)NR_2$, $SiR_3$, $(S{=}O)R$, $(S{=}O)_2R$, $(P{=}O)R_2$; wherein each R independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl, and

"a" is an integer from 0 to 2.

Surprisingly, it has been found that by using compounds of Formula (I) as electron transport material in an OLED device results in better OLED device performance. The compound of Formula (I) comprises a spiro[benzo[de]anthracene-7,9'-fluorene] structural unit. This unit facilitates sufficiently high glass transition temperature of the electron transport materials and sufficiently high charge carrier mobility at the same time to enable enhanced performance of an organic electronic device comprising such material of Formula (I). Using compounds of Formula (I) in an electron transport layer as neat material or as a host material in combination with an additive (metal, salt, complex) improves the device performance in particular with respect to lifetime and durability.

In the inventive organic electronic device, A may be unsubstituted. In this way, fine tuning of the electronic structure of the compound of Formula (I) can be achieved to further improve the usability thereof in organic semiconducting layers of organic electronic devices, in particular in electron transport layers.

In the inventive organic electronic device, in case that L is substituted, the respective substituents may be independently selected from the group consisting of phenyl, naphthyl, pyridyl, bi-phenylyl, dibenzofuranyl, dibenzothiophenyl and carbazolyl. In this way, fine tuning of the electronic structure of the compound of Formula (I) can be achieved to further improve the usability thereof in organic semiconducting layers of organic electronic devices, in particular in electron transport layers.

In the inventive organic electronic device, the L may be selected from the group consisting of triazine, pyrimidine, benzoacridine and dibenzoacridine. In this way, fine tuning of the electronic structure of the compound of Formula (I) can be achieved to further improve the usability thereof in organic semiconducting layers of organic electronic devices, in particular in electron transport layers.

In the inventive organic electronic device, in case that L is substituted, the substituents may be independently selected from the group consisting of

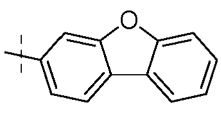 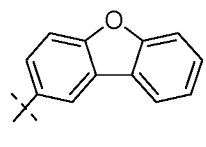 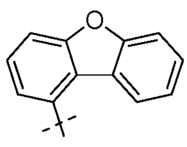 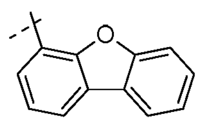

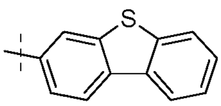 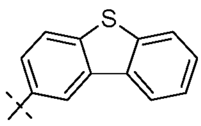 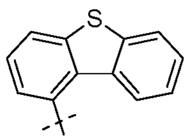 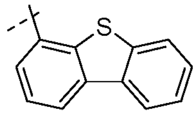

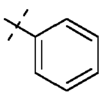 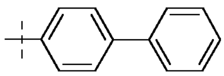 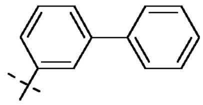 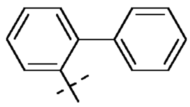

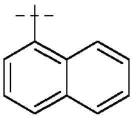 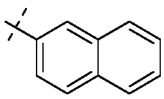 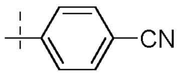 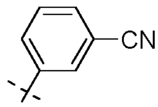

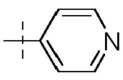 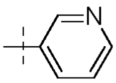 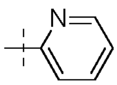 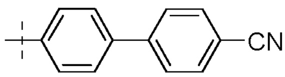 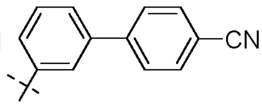

In this way, fine tuning of the electronic structure of the compound of Formula (I) can be achieved to further improve the usability thereof in organic semiconducting layers of organic electronic devices, in particular in electron transport layers.

In the inventive organic electronic device, the layer comprising the compound of Formula (I) may consist of at least one compound of Formula (I). This embodiment is particularly suitable to achieve an organic electronic device in which the layer comprising the compound of Formula (I) is a charge injection layer, respectively an electron injection layer.

Alternatively, in the inventive electronic device, the layer comprising the compound of Formula (I) may further comprise a metal, a metal salt or an organic metal complex, alternatively an alkali metal additive or a rare earth metal additive, alternatively a rare earth metal or an alkali metal complex or an alkali metal salt, alternatively Yb or LiQ or alkali borate or alkali phenolate, alternatively LiQ. This embodiment is suitable to achieve an organic electronic device in which the layer comprising the compound of Formula (I) is an electron transport layer.

The inventive organic electronic device may further comprise an emission layer, wherein the layer comprising the compound of Formula (I) is arranged between the emission layer and the cathode. Particularly good effects as to the improvement of performance of the organic electronic devcie were achieved in such an arrangement.

In the inventive organic electronic device, the device may further comprise an electron transport layer and the layer comprising the compound of Formula (I) is arranged between the electron transport layer and the cathode. Also in such an arrangement, particularly pronounced improvement of the device performance was observed.

The object is further achieved by a compound having the general Formula (I)

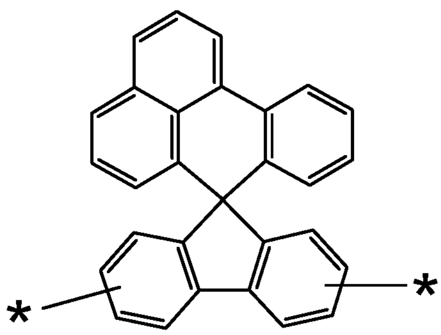

(I)

wherein

$$*-(A)_a-L$$

the compound of Formula (I) comprises one or more moieties -$(A)_a$-L and the remaining positions are hydrogen or substituents independently selected from the group consisting of deuterium, fluorine, RF, $C_1$-$C_{20}$ linear alkyl, $C_3$-$C_{20}$ branched alkyl, $C_1$-$C_{12}$ linear fluorinated alkyl, CN, RCN, $C_6$-$C_{20}$ aryl, $C_2$-$C_{20}$ heteroaryl, $(P{=}O)R_2$; wherein each R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl;

A is selected from substituted or unsubstituted $C_6$-$C_{12}$ aryl or $C_2$-$C_{20}$ heteroaryl; wherein in case that A is substituted, the respective substituents are independently selected from the group consisting of deuterium, fluorine, $C_1$-$C_{20}$ linear alkyl, $C_3$-$C_{20}$ branched alkyl, linear fluorinated $C_1$-$C_{12}$ alkyl, CN, $C_6$-$C_{20}$ aryl, and $C_2$-$C_{20}$ heteroaryl;

L is selected from the group consisting of triazine, pyrimidine, benzoacridine, dibenzoacridine, pyridine, bi-pyridine, benzimidazole, phenanthroline, benzo-nitrile, phenanthridine, benzooxazole, benzothiazole, phenanthridine-one, naphto-benzofurane, di-naphtofurane, benzo-naphto-thiophene, dinaphtothiophene, or a polar group selected from

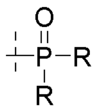 , 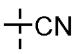 and 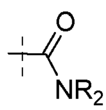 ,

wherein substituents, if present in the respective group L are independently selected from the group consisting of deuterium, fluorine, $C_1$-$C_{20}$ linear alkyl, $C_3$-$C_{20}$ branched alkyl, $C_3$-$C_{20}$ cyclic alkyl, $C_1$-$C_{20}$ linear alkoxy, $C_3$-$C_{20}$ branched alkoxy, $C_1$-$C_{12}$ linear fluorinated alkyl, $C_1$-$C_{12}$ linear fluorinated alkoxy, $C_3$-$C_{12}$ branched fluorinated cyclic alkyl, $C_3$-$C_{12}$ fluorinated cyclic alkyl, $C_3$-$C_{12}$ fluorinated cyclic alkoxy, CN, RCN, $C_6$-$C_{20}$ aryl, $C_2$-$C_{20}$ heteroaryl, OR, SR, (C=O)R, $(C{=}O)NR_2$, $SiR_3$, (S=O)R, $(S{=}O)_2R$, $(P{=}O)R_2$; wherein each R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl;

wherein in case that L is a N-containing heteroaryl group, the compound of the Formula (I) does only comprise one *-$(A)_a$-L moiety in which the $C_2$-$C_{42}$ heteroaryl is a N-containing heteroaryl group.

The compound of Formula (I) may have a HOMO energy level from -5.53 eV to -5.17 eV. A respective HOMO level was found to be particular advantageous for the use of the compounds of Formula (I) in organic electronic devices.

The compound of Formula (I) may have a LUMO energy level from -2.16 eV to -1.26 eV. A respective LUMO energy level was found to be particularly advantageous for using the inventive compounds of Formula (I) in organic electronic devices. A preferred LUMO energy level for use of the compounds of Formula (I) in an n-ETL together with an additive is from -2.1 eV to -1.8 eV. Furthermore, a LUMO energy level within the range from -1.9 to -1.7 eV was found to be particularly advantageous for use of the compounds of Formula (I) in hole blocking layers, particularly without the use of an additive.

The compound of Formula (I) may have a dipole moment from 0.14 Debye to 5.44 Debye. Such dipole moments were found to be particularly advantageous for use of the compounds of Formula (I) in organic electronic devices.

The compound of Formula (I) may be selected from the structures B-1 to B-46.

| Compound name | Structure | HOMO level (eV) | LUMO level (eV) | Dipole moment (Debye) |
|---|---|---|---|---|
| B-1 | 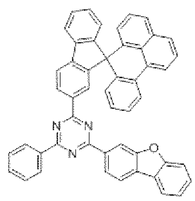 | -5.36 | -1.92 | 0.14 |

(continued)

| Compound name | Structure | HOMO level (eV) | LUMO level (eV) | Dipole moment (Debye) |
|---|---|---|---|---|
| B-2 | 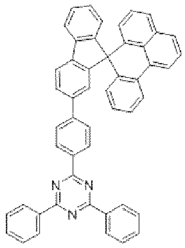 | -5.37 | -1.88 | 0.72 |
| B-3 | 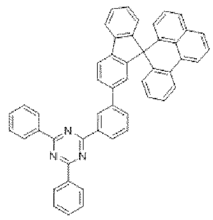 | -5.35 | -1.80 | 0.22 |
| B-4 | 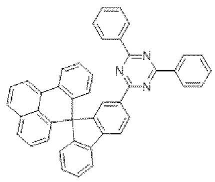 | -5,37 | -1,87 | 0,59 |
| B-5 | 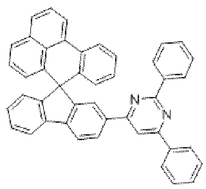 | -5,46 | -1,71 | 2,28 |
| B-6 | 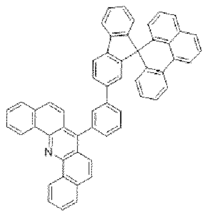 | -5,40 | -1,70 | 2,16 |
| B-7 | 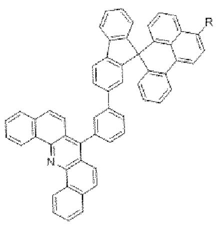 | -5,51 | -1,71 | 2,25 |
| B-8 | 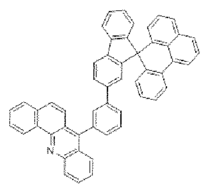 | -5,41 | -1,81 | 2,38 |

(continued)

| Compound name | Structure | HOMO level (eV) | LUMO level (eV) | Dipole moment (Debye) |
|---|---|---|---|---|
| B-9 | 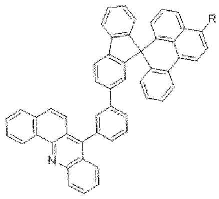 | -5,51 | -1,84 | 2,31 |
| B-10 | 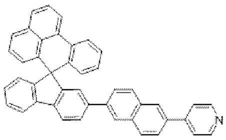 | -5,40 | -1,64 | 3,42 |
| B-11 | 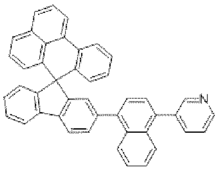 | -5,38 | -1,39 | 2,49 |
| B-12 | 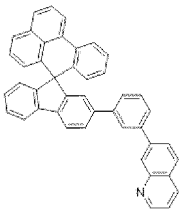 | -5,34 | -1,50 | 1,71 |
| B-13 | 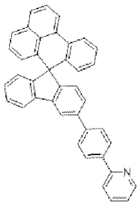 | -5,34 | -1,33 | 1,74 |
| B-14 | 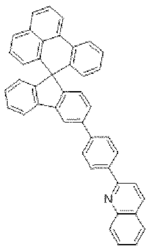 | -5,34 | -1,64 | 1,59 |
| B-15 | 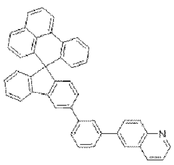 | -5,37 | -1,51 | 2,18 |

(continued)

| Compound name | Structure | HOMO level (eV) | LUMO level (eV) | Dipole moment (Debye) |
|---|---|---|---|---|
| B-16 | 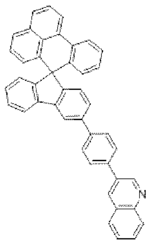 | -5,37 | -1,56 | 2,15 |
| B-17 | 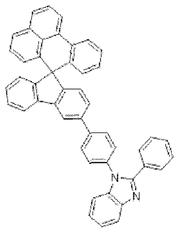 | -5,44 | -1,40 | 4,29 |
| B-18 | 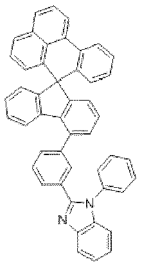 | -5,29 | -1,26 | 3,71 |
| B-19 | 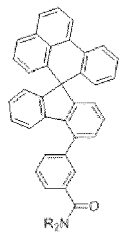 | -5,44 | -2,16 | 5,17 |
| B-20 | 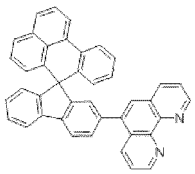 | -5,43 | -1,49 | 3,86 |
| B-21 | 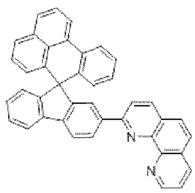 | -5,21 | -1,66 | 2,69 |

(continued)

| Compound name | Structure | HOMO level (eV) | LUMO level (eV) | Dipole moment (Debye) |
|---|---|---|---|---|
| B-22 | 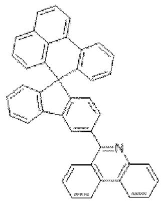 | -5,34 | -1,50 | 1,80 |
| B-23 | 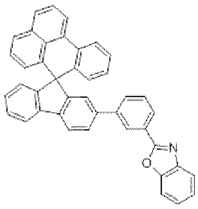 | -5,37 | -1,46 | 0,85 |
| B-24 | 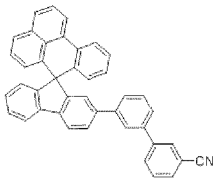 | -5,41 | -1,46 | 4,73 |
| B-25 | 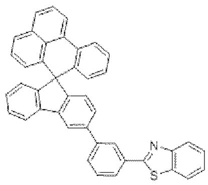 | -5,35 | -1,61 | 0,38 |
| B-26 | 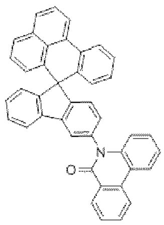 | -5,38 | -1,34 | 2,45 |
| B-27 | 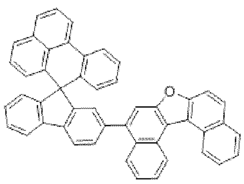 | -5,17 | -1,49 | 0,41 |
| B-28 | 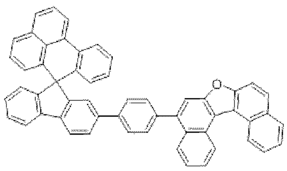 | -5,19 | -1,51 | 0,49 |

(continued)

| Compound name | Structure | HOMO level (eV) | LUMO level (eV) | Dipole moment (Debye) |
|---|---|---|---|---|
| B-29 | 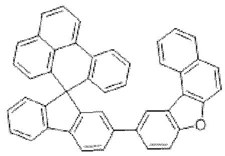 | -5,35 | -1,32 | 0,61 |
| B-30 | 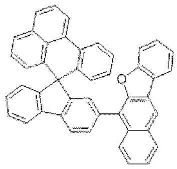 | -5,29 | -1,43 | 0,48 |
| B-31 | 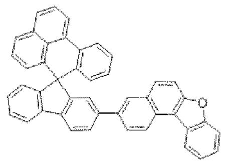 | -5,35 | -1,36 | 0,71 |
| B-32 | 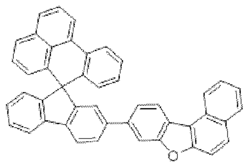 | -5,32 | -1,46 | 0,54 |
| B-33 | 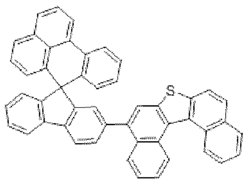 | -5,23 | -1,55 | 0,77 |
| B-34 | 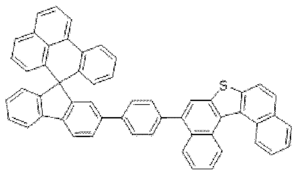 | -5,25 | -1,58 | 0,72 |
| B-35 | 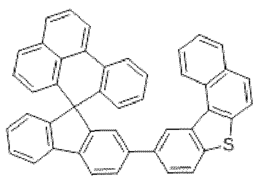 | -5,35 | -1,37 | 0,84 |
| B-36 | 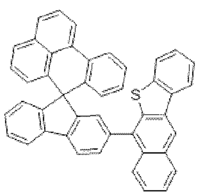 | -5,25 | -1,40 | 0,34 |

(continued)

| Compound name | Structure | HOMO level (eV) | LUMO level (eV) | Dipole moment (Debye) |
|---|---|---|---|---|
| B-37 | 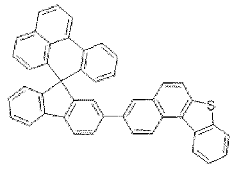 | -5,35 | -1,42 | 0,87 |
| B-38 | 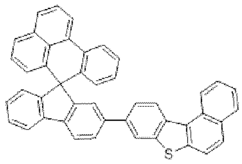 | -5,33 | -1,51 | 0,70 |
| B-39 | 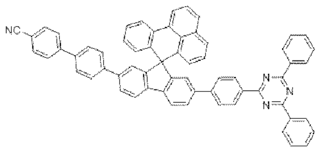 | -5,49 | -2,01 | 5,43 |
| B-40 | 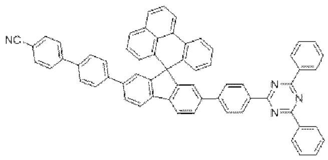 | -5,48 | -2,01 | 5,44 |
| B-41 | 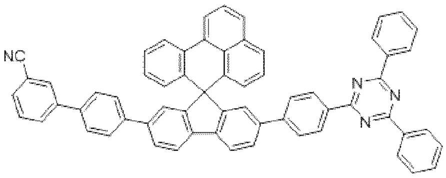 | -5,47 | -1,98 | 4,85 |
| B-42 | 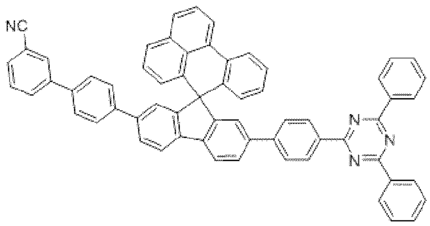 | -5,46 | -1,97 | 4,74 |
| B-43 | 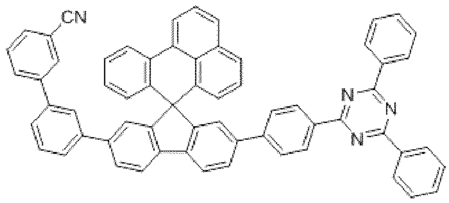 | -5,38 | -1,94 | 4,50 |
| B-44 | 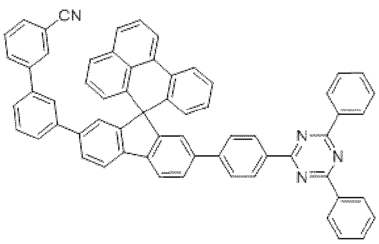 | -5,38 | -1,93 | 4,50 |

(continued)

| Compound name | Structure | HOMO level (eV) | LUMO level (eV) | Dipole moment (Debye) |
|---|---|---|---|---|
| B-45 | 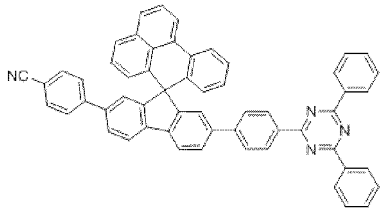 | -5,53 | -2,06 | 5,42 |
| B-46 | 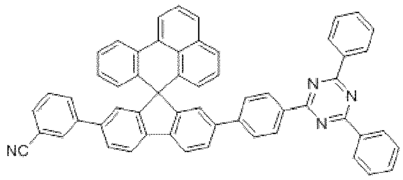 | -5,47 | -2,00 | 4,52 |

The HOMO and LUMO energy levels (eV) and dipole moments (Debye) for compounds of formula (1) were calculated using the program package TURBOMOLE V6.5 and the hybrid functional B3LYP with a 6-31G* basis set.
The object is further achieved by an organic semiconducting layer comprising the inventive compound.
The object is further achieved by a display device comprising the inventive organic electronic device.
Finally, the object is achieved by a lighting device comprising the inventive organic electronic device.

Further layers

In accordance with the invention, the organic electronic device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either the first electrode or the second electrode may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

The hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.
When the HIL is formed using spin coating or printing, coating conditions may vary according to the compound

that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may be a pure layer of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; $\alpha$-NPD (N,N'-Bis(naphthalen-1-yl)-N,N'-bis(phenyl)-benzidine) doped with F4TCNQ. $\alpha$-NPD doped with 2,2'-(perfluoronaphthalen-2,6-diylidene) dimalononitrile. $\alpha$-NPD doped with 2,2',2"-(cyclopropane-1,2,3-triylidene)tris(2-(p-cyanotetrafluorophenyl)acetonitrile). Dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of the electron blocking layer (EBL) is to prevent electrons from being transferred from the emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconducting layer comprising the inventive compound represented by the general Formula (I) as defined above.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include xadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

*Electron transport layer (ETL)*

The OLED according to the present invention may contain an electron transport layer (ETL). In accordance with the invention, the electron transport layer may be the inventive organic semiconducting layer comprising the inventive compound represented by the general Formula (I) as defined above.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer of the organic electronic device may comprise the compound represented by general Formula (I) as defined above as the organic electron transport matrix (ETM) material. The electron transport layer may comprise, besides the compound represented by the general Formula (I), further ETM materials known in the art. Likewise, the electron transport layer may comprise as the only electron transport matrix material the compound represented by general Formula (I). In case that the inventive organic electronic device comprises more than one electron transport layers, the compound represented by the general Formula (I) may be comprised in only one of the electron transport layers, in more than one of the electron transport layers or in all of the electron transport layers. In accordance

with the invention, the electron transport layer may comprise, besides the ETM material, at least one additive as defined below. Further, the electron transport layer may comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (I) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

*Electron injection layer (EIL)*

The optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may be the layer comprising the compound of Formula (I).

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode electrode*

The cathode electrode is formed on the EIL if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

*Charge generation layer/hole generating layer*

The charge generation layer (CGL) may be composed of a double layer.

Typically, the charge generation layer is a pn junction joining a n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem devices, for example, in tandem OLEDs comprising, between two electrodes, two or more emission layers. In a tandem OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB).

The n-type charge generating layer may be the layer comprising the compound of Formula (I). The n-type charge generation layer can be layer of a neat n-type dopant, for example of an electropositive metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

In one embodiment, the n-type charge generation layer may include compounds of the following Chemical Formula X.

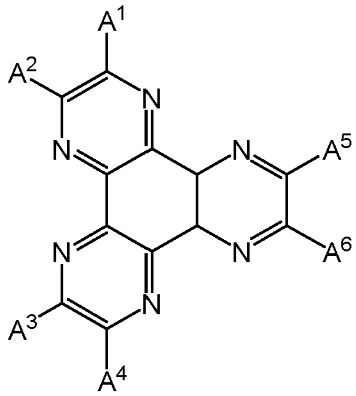

, (X),

wherein each of $A^1$ to $A^6$ may be hydrogen, a halogen atom, nitrile (-CN), nitro (-NO2), sulfonyl (-SO2R), sulfoxide (-SOR), sulfonamide (-SO2NR), sulfonate (-SO3R), trifluoromethyl (-CF3), ester (-COOR), amide (-CONHR or -CONRR'), substituted or unsubstituted straight-chain or branched-chain C1-C12 alkoxy, substituted or unsubstituted straight-chain or branched-chain C1-C12 alkyl, substituted or unsubstituted straight-chain or branched chain C2-C12 alkenyl, a substituted or unsubstituted aromatic or non-aromatic heteroring, substituted or unsubstituted aryl, substituted or unsubstituted mono- or di-arylamine, substituted or unsubstituted aralkylamine, or the like. Herein, each of the above R and R' may be substituted or unsubstituted C1-C60 alkyl, substituted or unsubstituted aryl, or a substituted or unsubstituted 5- to 7-membered heteroring, or the like.

An example of such n-type charge generation layer may be a layer comprising CNHAT

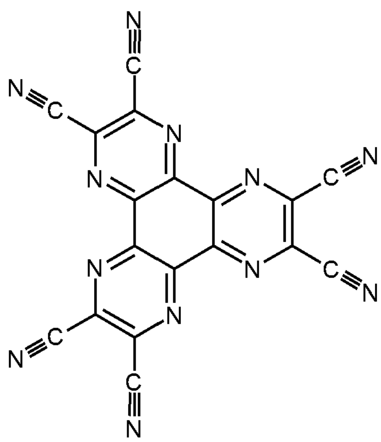

(CNHAT).

The hole generating layer is arranged on top of the n-type charge generation layer.

*Organic light-emitting diode (OLED)*

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer, and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer, an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

For example, the OLED according to Fig. 2 may be formed by a process, wherein
on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

<u>Organic electronic device</u>

An organic electronic device according to the invention comprises an organic semiconducting layer comprising a compound according to Formula I.

An organic electronic device according to one embodiment may include a substrate, an anode layer, an organic semiconducting layer comprising a compound of Formula (I) and a cathode layer.

An organic electronic device according to one embodiment comprises at least one organic semiconducting layer comprising at least one compound of Formula I, at least one anode layer, at least one cathode layer and at least one emission layer, wherein the organic semiconducting layer is preferably arranged between the emission layer and the cathode layer.

An organic light-emitting diode (OLED) according to the invention may include an anode, a hole transport layer (HTL), an emission layer (EML), an electron transport layer (ETL) comprising at least one compound of Formula (I), and a cathode, which are sequentially stacked on a substrate. In this regard, the HTL, the EML, and the ETL are thin films formed from organic compounds.

An organic electronic device according to one embodiment can be a light emitting device, thin film transistor, a battery, a display device or a photovoltaic cell, and preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of Formula (I) according to the invention, and
- a second deposition source to release the alkali halide or alkali organic complex, preferably a lithium halide or lithium organic complex;

the method comprising the steps of forming the electron transport layer stack; whereby for an organic light-emitting diode (OLED):

- the first electron transport layer is formed by releasing the compound of Formula (I) according to the invention from the first deposition source and an alkali metal compound, preferably an alkali halide or alkali organic complex, preferably a lithium halide or lithium organic complex from the second deposition source.

According to various embodiments of the present invention, the method may further include forming on the anode electrode an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, preferably a first electron transport layer is formed on the emission layer and optional a second electron transport layer is formed,
- and finally a cathode electrode is formed,
- optional a hole injection layer, a hole transport layer, and a hole blocking layer, formed in that order between the first anode electrode and the emission layer,
- optional an electron injection layer is formed between the electron transport layer and the cathode electrode.

According to various embodiments of the present invention, the method may further include forming an electron injection layer on a first electron transport layer. However, according to various embodiments of the OLED of the present invention, the OLED may not comprise an electron injection layer.

According to various embodiments, the OLED may have the following layer structure, wherein the layers having the following order:
anode, hole injection layer, first hole transport layer, second hole transport layer, emission layer, optional second electron transport layer, first electron transport layer comprising a compound of Formula (I) according to the invention, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconducting layer comprising a compound according to Formula (I) can further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

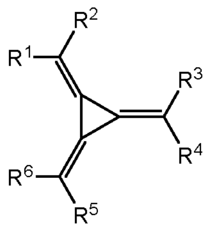 (XX) 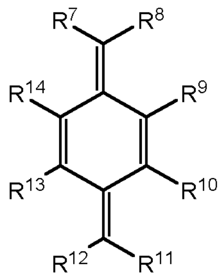 (XXIa) 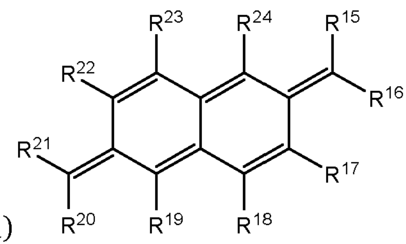 (XXIb),

wherein $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

Hereinafter, the embodiments are illustrated in more detail with reference to examples. However, the present disclosure is not limited to the following examples. Reference will now be made in detail to the exemplary aspects.

Details and definitions of the invention

In the Formula (I) the " * " marks the possible positions to which the group-$(A)_a$-L may be bound. In each of the aromatic rings labeled with the "*", the -$(A)_a$-L moiety may be bound in each position not already having four bindings. The respective binding is by replacing a hydrogen by the respective moiety. In this regard, it may be provided that the compound of Formula (I) comprises only one -$(A)_a$-L moiety. Likewise, it may be provided that the compound of Formula (I) comprises more than one -$(A)_a$-L moiety unless explicitly mentioned else. In this regard, it is provided that the -$(A)_a$-L moiety is only attached to the rings labeled accordingly and that the remaining aromatic rings do not comprise a respective -$(A)_a$-L moiety.

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

The subscribed number n in $C_n$ relates to the total number of carbon atoms in the respective alkyl, arylene, heteroarylene or aryl group.

The term "aryl" as used herein shall encompass, unless explicitly mentioned else, phenyl ($C_6$-aryl) or fused aromatics, such as naphthalene, anthracene, phenanthracene, tetracene etc. Further encompassed shall be any further aromatic hydrocarbon substituents, such as fluorenyl etc. As used herein, the term "aryl" is also used for "arylene" moieties. For example, the group A, which may be an aryl group, is bound to two different moieties, namely the spiro part of Formula (I) and the moiety L. In the present specification the term "aryl group" may refer to a group comprising at least one hydrocarbon aromatic moiety, and all the elements of the hydrocarbon aromatic moiety may have p-orbitals which form conjugation, for example a phenyl group, a naphtyl group, an anthracenyl group, a phenanthrenyl group, a pyrenyl group, a fluorenyl group and the like. The arylene group may include a monocyclic or fused ring polycyclic (i.e., rings sharing adjacent pairs of carbon atoms) functional group.

The term "heteroaryl" as used herein refers to aryl groups in which at least one carbon atom is substituted with a heteroatom, preferably selected from N, O, S, B or Si.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms.

Preferably a heteroarylene ring may comprise at least 1 to 3 heteroatoms individually selected from N, S and/or O.

The term "heteroaryl" as used herewith shall encompass pyridine, quinoline, quinazoline, pyridine, triazine, benzimidazole, benzothiazole, benzo[4,5]thieno[3,2-d]pyrimidine, carbazole, xanthene, phenoxazine, benzoacridine, dibenzoacridine and the like.

In the present specification, the single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

With respect to the inventive organic semiconductive layer as well as with respect to the inventive compound, the compounds mentioned in the experimental part are most preferred.

The inventive organic electronic device may be an organic electroluminescent device (OLED) an organic photovoltaic device (OPV), a lighting device, or an organic field-effect transistor (OFET). A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention may comprise more than one emission layer, preferably two or three emission layers. An OLED comprising more than one emission layer is also described as a tandem OLED or stacked OLED.

The organic electroluminescent device (OLED) may be a bottom- or top-emission device.

Another aspect is directed to a device comprising at least one organic electroluminescent device (OLED). A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The energy levels of the highest occupied molecular orbital, also named HOMO, and of the lowest unoccupied molecular orbital, also named LUMO, are measured in electron volt (eV) indirectly by cyclic voltammetry vs ferrocene or can be calculated Such DFT calculations may be carried out using the program package TURBOMOLE V6.5 (Provider: TURBOMOLE GmbH, Litzenhardtstrasse 19, 76135 Karlsruhe, Germany). The optimized geometries and the HOMO/LUMO energy levels of the molecular structures are determined using the hybrid functional B3LYP with a 6-31G* basis set. If more than one conformation is viable, the conformation with the lowest total energy is selected.The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of', "does not contain", "does not comprise" does not exclude impurities. Impurities have no technical effect with respect to the object achieved by the present invention.

In the context of the present specification the term "essentially non-emissive" or "non-emitting" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the organic semiconducting layer comprising the compound of Formula I is essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "transition metal" means and comprises any element in the d-block of the periodic table, which comprises groups 3 to 12 elements on the periodic table.

The term "group III to VI metal" means and comprises any metal in groups III to VI of the periodic table.

The term "life-span" and "lifetime" are simultaneously used and have the same meaning.

All numeric values are herein assumed to be modified by the term "about", whether or not explicitly indicated. As used herein, the term "about" refers to variation in the numerical quantity that can occur.

Whether or not modified by the term "about" the claims include equivalents to the quantities.

It should be noted that, as used in this specification and the appended claims, the singular forms "a", "an", and "the" include plural referents unless the content clearly dictates otherwise.

The anode electrode and cathode electrode may be described as anode electrode / cathode electrode or anode electrode / cathode electrode or anode electrode layer / cathode electrode layer.

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom in the molecule.

The dipole moment is determined by a semi-empirical molecular orbital method.

The partial charges and atomic positions in the gas phase are obtained using the hybrid functional B3LYP with a 6-31G* basis set as implemented in the program package TURBOMOLE V6.5. If more than one conformation is viable, the conformation with the lowest total energy is selected to determine the dipole moment.

The reduction potential may be determined by cyclic voltammetry with potentiostatic device Metrohm PGSTAT30 and software Metrohm Autolab GPES at room temperature. The redox potentials are measured in an argon de-aerated, anhydrous 0.1M THF solution of the compound of Formula I, under argon atmosphere, with 0.1M tetrabutylammonium hexafluorophosphate as supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run is done in the broadest range of the potential set on the working electrodes, and the range is then adjusted within subsequent runs appropriately. The final three runs are done with the addition of ferrocene (in 0.1M concentration) as the standard. The average of potentials corresponding to cathodic and anodic peak of the compound is determined through subtraction of the average of cathodic and anodic potentials observed for the standard $Fc^+/Fc$ redox couple.

Room temperature, also named ambient temperature, is 23°C.

BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED), according to an exemplary embodiment of the present invention;

FIG. 2 is a schematic sectional view of an OLED, according to an exemplary embodiment of the present invention.

FIG. 3 is a schematic sectional view of a tandem OLED comprising a charge generation layer, according to an exemplary embodiment of the present invention.

DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Instead of a single electron transport layer 160, optionally an electron transport layer stack (ETL) can be used.

Fig. 2 is a schematic sectional view of an OLED 100, according to another exemplary embodiment of the present invention. Fig. 2 differs from Fig. 1 in that the OLED 100 of Fig. 2 comprises an electron blocking layer (EBL) 145 and a hole blocking layer (HBL) 155.

Referring to Fig. 2, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

Fig. 3 is a schematic sectional view of a tandem OLED 200, according to another exemplary embodiment of the present invention. Fig. 3 differs from Fig. 2 in that the OLED 100 of Fig. 3 further comprises a charge generation layer (CGL) and a second emission layer (151).

Referring to Fig. 3, the OLED 200 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

While not shown in Fig. 1, Fig. 2 and Fig. 3, a sealing layer may further be formed on the cathode electrodes 190, in order to seal the OLEDs 100 and 200. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

The features disclosed in the foregoing description, in the claims and/or in the accompanying drawings may, both separately and in any combination thereof, be material for realizing the invention in diverse forms thereof.

EXPERIMENTAL PART

**Experimental data**

Synthesis of compound of Formula 1

**2'-bromospiro[benzo[*de*]anthracene-7,9'-fluorene]** was synthesized following literature procedure (J.-Y. Kim et al., Dyes and Pigments **2012,** *94,* 304-313).

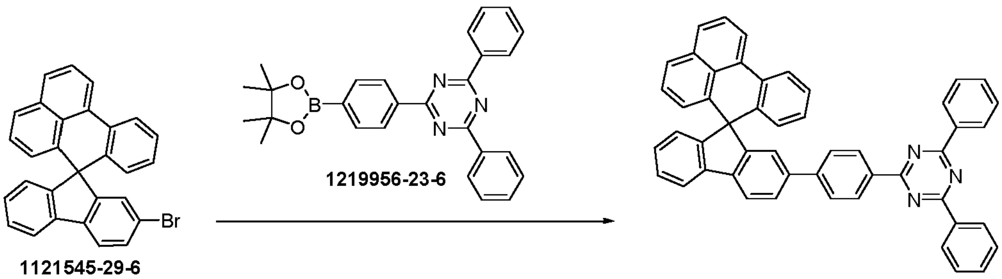

1121545-29-6

1219956-23-6

**2,4-diphenyl-6-(4-(spiro[benzo[*de*]anthracene-7,9'-fluoren]-2'-yl)phenyl)-1,3,5-triazine**

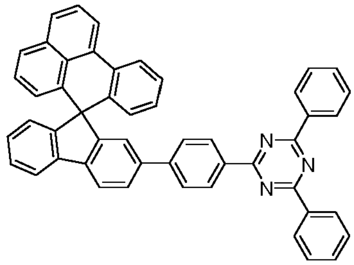

A flask was flushed with nitrogen and charged with 2'-bromospiro[benzo[de]anthracene-7,9'-fluorene] (5.1 g, 11.4 mmol), 2,4-diphenyl-6-(4-(4,4,5,5-tetramethyl-1,3,2-dioxaborolan-2-yl)phenyl)-1,3,5-triazine (4.9 g, 11.4 mmol), $K_2CO_3$ (4.7 g, 34.3 mmol), and $Pd(PPh_3)_4$ (0.39 g, 0.3 mmol). A mixture of deaerated 1,4-dioxane/water (6:1, 127 mL) was added and the reaction mixture was heated to 95 °C under a nitrogen atmosphere for 1.5 h. Subsequently, the formed precipitate was collected by suction filtration and washed with 1,4-dioxane. The solid was dissolved in hot chlorobenzene and filtered through a pad of silica gel. The filtrate was concentrated under reduced pressure, and the residue recrystallized from a mixture of chloroform/n-hexane. After drying in vacuo, 4.12 g (53%) of a white solid were obtained. Final purification was achieved by sublimation. HPLC/ESI-MS: 100%, m/z = 674 ($[M+H]^+$).

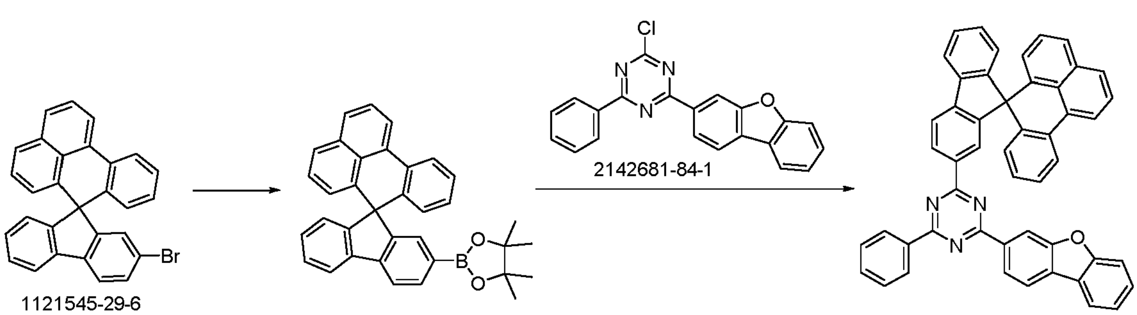

1121545-29-6

2142681-84-1

**4,4,5,5-tetramethyl-2-(spiro[benzo[*de*]anthracene-7,9'-fluoren]-2'-yl)-1,3,2-dioxaborolane**

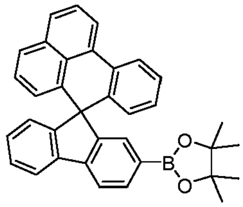

A flask was flushed with nitrogen and charged with 2'-bromospiro[benzo[*de*]anthracene-7,9'-fluorene] (6.4 g, 14.3 mmol), bis(pinacolato)diboron (4.0 g, 15.0 mmol), potassium acetate (3.5 g, 35.2 mmol), and Pd(dppf)$Cl_2$ (0.52 g, 0.7 mmol). Deaerated DMF (65 mL) was added and the reaction mixture was heated to 80 °C under a nitrogen atmosphere for 17 h. After cooling down to room temperature the solvent was removed under reduced pressure, the residue was dissolved in dichloromethane and the organic layer washed with water, dried over $MgSO_4$, and filtered through a pad of Florisil. The filtrate was concentrated under reduced pressure, the residue was triturated with hot n-hexane, and the formed solid was recovered by suction filtration. After drying in vacuuo 5.0 g (70%) of an off-white solid were obtained. HPLC: 99%.

**2-(dibenzo[*b,d*]furan-3-yl)-4-phenyl-6-(spiro[benzo[*de*]anthracene-7,9'-fluoren]-2'-yl)-1,3,5,-triazine**

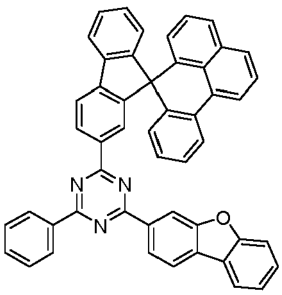

_A flask was flushed with nitrogen and charged with 2-chloro-4-(dibenzo[*b,d*]furan-3-yl)-6-phenyl-1,3,5-triazine (3.2 g, 8.9 mmol), 4,4,5,5-tetramethyl-2-(spiro[benzo[de]anthracene-7,9'-fluoren]-2'-yl)-1,3,2-dioxaborolane (4.8 g, 9.8 mmol), ), $K_2CO_3$ (3.7 g, 26.8 mmol), and $Pd(PPh_3)_4$ (0.31 g, 0.2 mmol). A mixture of deaerated 1,4-dioxane/water (4:1, 67 mL) was added and the reaction mixture was heated to 90 °C under a nitrogen atmosphere for 18 h. After cooling down to room temperature the formed precipitate was collected by suction filtration and washed with water and methanol. The solid was dissolved in dichloromethane, and the organic layer was dried over $Na_2SO_4$ and filtered through a pad of Florisil. The filtrate was concentrated under reduced pressure, the residue was triturated with n-hexane, and the formed solid was recovered by suction filtration, and recrystallized from a mixture of n-hexane/methanol. After drying in vacuo, 3.5 g (57%) of a white solid were obtained. Final purification was achieved by sublimation. HPLC/ESI-MS: 100%, m/z = 688 ($[M+H]^+$).

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Reduction potential

The reduction potential is determined by cyclic voltammetry with potentiostatic device Metrohm PGSTAT30 and software Metrohm Autolab GPES at room temperature. The redox potentials given at particular compounds were

measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behaviour.

Dipole moment, LUMO level. HOMO level

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set as implemented in the program package TURBOMOLE V6.5. If more than one conformation is viable, the conformation with the lowest total energy is selected to determine the bond lengths of the molecules.

DFT calculations using the program package TURBOMOLE V6.5 are also used in conjunction with the optimized molecular geometries to determine the HOMO and LUMO energy levels of the molecular structures using the hybrid functional B3LYP with a 6-31G* basis set. If more than one conformation is viable, the conformation with the lowest total energy is selected.

Provider: TURBOMOLE GmbH, Litzenhardtstrasse 19, 76135 Karlsruhe, Germany

General procedure for fabrication of OLEDs

For top emission OLED devices, inventive examples and comparative examples, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode. 100 nm Ag were deposited as anode at a pressure of 10-5 to 10-7 mbar to form the anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the ITO electrode, to form a HIL having a thickness of 10 nm. Then, Biphenyl-4-yl(9,9-diphenyl-9H-fluoren-2-yl)-[4-(9-phenyl-9H-carbazol-3-yl)phenyl]-amine was vacuum deposited on the HIL, to form a HTL having a thickness of 118 nm.

Then, N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then, the emission layer was deposited. 97 vol.-% H09 (Fluorescent-blue host material, Sun Fine Chemicals) as EML host and 3 vol.-% BD200 (Sun Fine Chemicals) as fluorescent blue dopant were deposited on the EBL, to form a blue-emitting EML with a thickness of 20 nm.

Then, for top emission OLED devices of configuration A, the hole blocking layer was deposited on the emission layer with a thickness of 5 nm. For example-1a compound of Formula 1 was deposited on the emission layer, according to table 4. For comparative example-1 the comparative compound-1 was deposited on the emission layer, according to table 4.

Then, the electron transporting layer is formed on the hole blocking layer with a thickness of 31 nm by co-deposition of 2-([1,1'-biphenyl]-4-yl)-4-(9,9-diphenyl-9H-fluoren-4-yl)-6-phenyl-1,3,5-triazine (CAS 1801992-44-8) and lithium quinolate (LiQ) in a wt% ratio of 1:1.

For top emission OLED devices of configuration B 2,4-diphenyl-6-(4',5',6'-triphenyl-[1,1':2',1":3",1''':3''',1''''-quinquephenyl]-3''''-yl)-1,3,5-triazine (CAS 2032364-64-8) was deposited as the hole blocking layer with a thickness of 5 nm on the emission layer. Then, for example-2 and example-3 a compound of Formula 1 was co-deposited with lithium quinolate (LiQ), according to table 5, in a wt% ratio of 1:1 on the emission layer to form the electron transporting layer with a thickness of 31 nm. For comparative example-2 the comparative compound-1 was co-deposited with lithium quinolate (LiQ), according to table 5, in a wt% ratio of 1:1 on the emission layer to form the electron transporting layer with a thickness of 31 nm.

Then, for both top emission OLED devices of configuration A and B the electron injection layer is formed on the electron transporting layer by deposing Yb with a thickness of 2 nm.

Ag is evaporated at a rate of 0.01 to 1 Å/s at $10^{-7}$ mbar to form a cathode with a thickness of 11 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 75 nm.

Overview of compounds used (non-inventive and non-comparative)

| IUPAC name | Reference |
|---|---|
| Biphenyl-4-yl(9,9-diphenyl-9H-fluoren-2-yl)-[4-(9-phenyl-9H-carbazol-3-yl)phenyl]-amine (CAS 1242056-42-3) | US2016322581 |
| 4,4',4"-((1E,1'E,1"E)-cyclopropane-1,2,3-triylidenetris (cyanomethanylylidene))tris(2,3,5,6-tetrafluorobenzonitrile) | US2008265216 |
| N,N-bis(4-(dibenzo[b,d]furan-4-yl)phenyl)-[1,1':4',1"-terphenyl]-4-amine (CAS 1198399-61-9) | JP2014096418 |
| H09 (Fluorescent-blue host material) | Commercially available from Sun Fine Chemicals, Inc, S. Korea |
| BD200 (Fluorescent-blue emitter material) | Commercially available from Sun Fine Chemicals, Inc, S. Korea |
| 2,4-diphenyl-6-(4',5',6'-triphenyl-[1,1':2',1":3",1''':3''',1''''-quinquephenyl]-3''''-yl)-1,3,5-triazine (CAS 2032364-64-8) | WO2016171358 |
| 2-([1,1'-biphenyl]-4-yl)-4-(9,9-diphenyl-9H-fluoren-4-yl)-6-phenyl-1,3,5-triazine (CAS 1801992-44-8) | KR101537500 |
| 8-Hydroxyquinolinolato-lithium (850918-68-2) = LiQ | WO2013079217 |

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristics are determined using a Keithley 2635 source measure unit by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in $cd/m^2$ using an Instrument Systems CAS-140CT array spectrometer, which has been calibrated by Deutsche Akkreditierungsstelle (DAkkS) for each of the voltage values. The cd/A efficiency at 10 $mA/cm^2$ is determined by interpolating the luminance-voltage and current-voltage characteristics, respectively.

In top emission devices, the emission is forward-directed, non-Lambertian and also highly dependent on the micro-cavity. Therefore, the external quantum efficiency (EQE) and power efficiency in lm/W will be higher compared to bottom emission devices.

**Technical Effect of the invention**

Material property

The Tg values are in a range suitable for use in organic electronic devices. Higher Tg values of materials used in organic electronics are generally preferred for device durability and robustness. The Tg of compound B-1 is significantly

increased over the Tg of the comparative compound-i.

Table 3 shows that the HOMO and LUMO energy levels and the dipole moments of compound B-1 of Formula 1 and of the comparative compound-i. The values are in a range suitable for use as hole blocking materials or electron transporting materials in organic electronic devices.

Top emission devices

Surprisingly, the cd/A (/ y) efficiency of top emission OLED devices is increased when using compounds of Formula 1 (instead of the comparative compound) as hole blocking layer. At the same time, the operating voltage of top emission OLED devices is decreased when using compounds of Formula 1 as a hole blocking layer instead of the comparative compound.

Surprisingly, the lifetime of top emission OLED devices is increased when using compounds of Formula 1 in an electron transport layer comprising a 1:1 wt% mixture of a compound of Formula 1 and LiQ.

Table 4 shows the operating voltage and cd/A (/ y) efficiencies of top emission OLED devices comprising a hole blocking layer comprising a compound of Formula 1. Table 5 shows the operating voltage and lifetime of top emission OLED devices comprising an electron transport layer comprising a 1:1 wt% mixture of a compound of Formula 1 and LiQ.

In summary, improved performance of top emission OLED devices can be achieved by using compounds of Formula 1.

**Experimental data (overview)**

**Table 1: Properties of comparative compounds**

| Compound name | Structure | Tg (°C) | Tm (°C) | TRO (°C) |
|---|---|---|---|---|
| Comparative compound-1 | 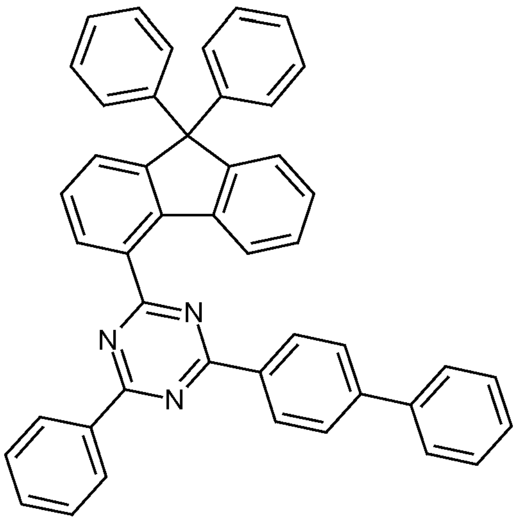 | 131 | 234 | 207 |

**Table 2: Properties of compounds of Formula 1**

| Compound of Formula 1 | Structure | Tg (°C) | Tm (°C) | TRO (°C) |
|---|---|---|---|---|
| Compound B-1 | 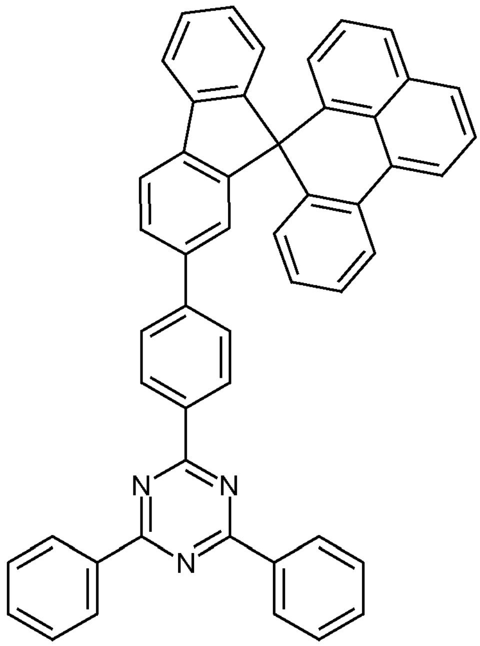 | 161 | 318 | 284 |
| Compound B-2 | 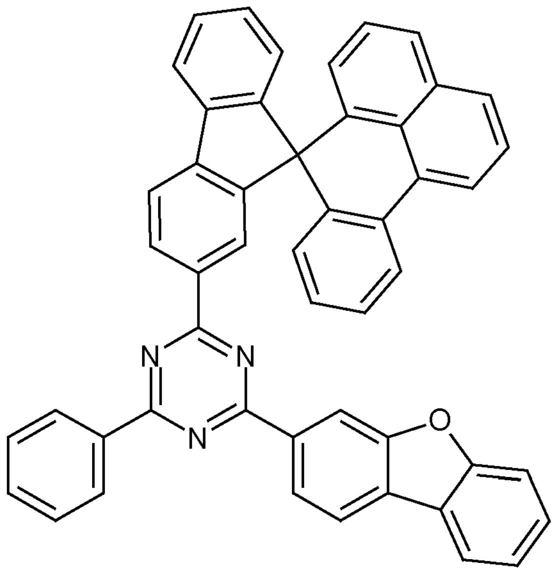 | 170 | 297 | 262 |

**Table 3: Energy levels and dipole moments of comparative compound and compounds of Formula 1**

| Compound name | HOMO (eV)* | LUMO (eV)* | Dipole moment (Debye)* |
|---|---|---|---|
| Comparative compound-1 | -5.81 | -1.89 | 0.64 |
| Compound B-1 | -5.37 | -1.88 | 0.72 |
| Compound B-2 | -5.36 | -1.92 | 0.14 |
| * Values calculated with B3LYP_Gaussian / 6-31G*, gas phase | | | |

Table 4: Operating voltage of top emission organic electroluminescent devices comprising a compound of Formula 1 in the hole blocking layer (configuration A).

| | Material of Hole blocking layer | Thickness hole blocking layer (nm) | CIEy | Operating voltage (V) at 10 mA/cm$^2$ | Ceff/CIEy (cd/A / y) at 0.045 |
|---|---|---|---|---|---|
| Comparative example 1 | Comparative compound 1 | 5 | 0.045 | 3.83 | 131 |
| Example 1 | Compound B-1 | 5 | 0.038 | **3.74** | **137** |

Table 5: Operating voltage of top emission organic electroluminescent devices comprising a 1:1 wt% mixture of compound of Formula 1 with LiQ in the electron transport layer (configuration B).

| | Material of electron transport layer | Mixing ratio (wt-%) with LiQ | Thickness electron transport layer (nm) | CIEy | Operating voltage at 10 mA/cm$^2$ (V) | Lifetime (97%) (hours) at 30mA / cm2 |
|---|---|---|---|---|---|---|
| Comparative example 2 | Comparative compound 1 | 1:1 | 31 | 0.044 | 3.47 | 29 |
| Example 2 | Compound B-1 | 1:1 | 31 | 0.046 | **3-45** | **46** |
| Example 3 | Compound B-2 | 1:1 | 31 | 0.046 | **3.41** | **51** |

## Claims

**1.** Organic electronic device comprising, between an anode and a cathode, at least one layer selected from an electron injection layer, an electron transport layer or an electron generation layer, the layer comprising at least one compound of the following Formula (I):

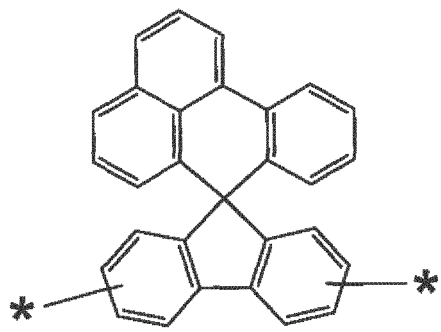

(I)

wherein

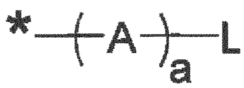

the compound of Formula (I) comprises one or more moieties $-(A)_a-L$ and the remaining positions marked with "*" are hydrogen or substituents independently selected from the group consisting of deuterium, fluorine, RF, $C_1$-$C_{20}$ linear alkyl, $C_3$-$C_{20}$ branched alkyl, $C_1$-$C_{12}$ linear fluorinated alkyl, CN, RCN, $C_6$-$C_{20}$ aryl, $C_2$-$C_{20}$ heteroaryl, $(P{=}O)R_2$; wherein each R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl;

A is selected from substituted or unsubstituted $C_6$-$C_{24}$ aryl or $C_2$-$C_{20}$ heteroaryl; wherein in case that A is substituted, the respective substituents are independently selected from the group consisting of deuterium, fluorine, $C_1$-$C_{20}$ linear alkyl, $C_3$-$C_{20}$ branched alkyl, linear fluorinated $C_1$-$C_{12}$ alkyl, CN, $C_6$-$C_{20}$ aryl, and $C_2$-$C_{20}$

heteroaryl;
L is selected from the group consisting of triazine, pyrimidine, benzoacridine, dibenzoacridine, pyridine, bi-pyridine, benzimidazole, phenanthroline, benzo-nitrile, phenanthridine, benzooxazole, benzothiazole, phenanthridine-one, naphto-benzofurane, di-naphtofurane, benzo-naphto-thiophene, dinaphtothiophene. or a polar group selected from

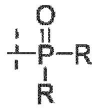 , 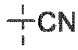 and 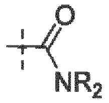 ,

wherein substituents, if present in the respective group L are independently selected from the group consisting of deuterium, fluorine, $C_1$-$C_{20}$ linear alkyl, $C_3$-$C_{20}$ branched alkyl, $C_3$-$C_{20}$ cyclic alkyl, $C_1$-$C_{20}$ linear alkoxy, $C_3$-$C_{20}$ branched alkoxy, $C_1$-$C_{12}$ linear fluorinated alkyl, $C_1$-$C_{12}$ linear fluorinated alkoxy, $C_3$-$C_{12}$ branched fluorinated cyclic alkyl, $C_3$-$C_{12}$ fluorinated cyclic alkyl, $C_3$-$C_{12}$ fluorinated cyclic alkoxy, CN, RCN, $C_6$-$C_{20}$ aryl, $C_2$-$C_{20}$ heteroaryl, OR, SR, (C=O)R, (C=O)$NR_2$, $SiR_3$, (S=O)R, $(S=O)_2R$, $(P=O)R_2$; wherein each R independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl, and
"a" is an integer from 0 to 2.

**2.** Organic electronic device according to claim 1, wherein A is unsubstituted.

**3.** Organic electronic device according to claim 1 or 2, wherein in case that L is substituted, the respective substituents are independently selected from the group consisting of phenyl, naphthyl, pyridyl, bi-phenylyl, dibenzofuranyl, dibenzothiophenyl and carbazolyl.

**4.** Organic electronic device according to any of the preceding claims, wherein L is selected from the group consisting of triazine, pyrimidine, benzoacridine and dibenzoacridine.

**5.** Organic electronic device according to any of the preceding claims, wherein, in case that L is substituted, the substituents are independently selected from the group consisting of

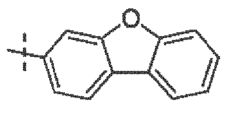 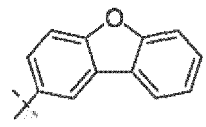 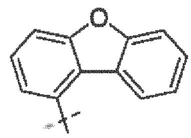 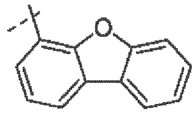

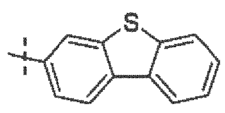 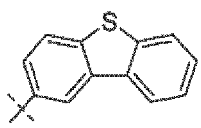 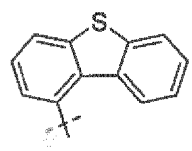 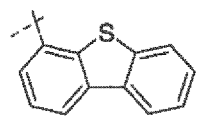

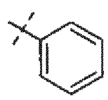 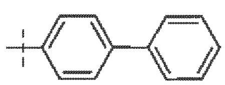 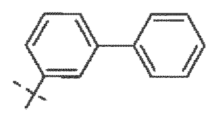 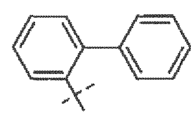

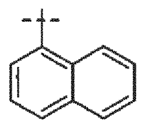 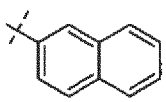 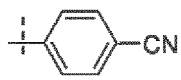 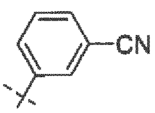

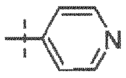 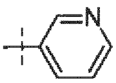 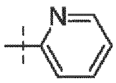 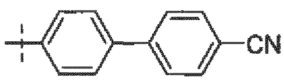 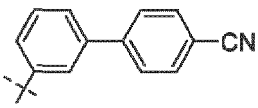

6. Organic electronic device according to any of the preceding claims, wherein the layer comprising the compound of Formula (I) consists of at least one compound of Formula (I).

7. Organic electronic device according to any of the preceding claims, wherein the layer comprising the compound of Formula (I) further comprises a metal, metal salt or organic metal complex, preferably an alkali metal additive or a rare earth metal additive, more preferred a rare earth metal or an alkali metal complex or an alkali metal salt, more preferably Yb or LiQ or alkali borate or alkali phenolate, more preferable LiQ.

8. Organic semiconducting device according to any of the preceding claims, wherein the organic electronic device further comprises an emission layer and the layer comprising the compound of Formula (I) is arranged between the emission layer and the cathode.

9. Organic semiconducting device according to any of the preceding claims, further comprising an electron transport layer, wherein the layer comprising the compound of Formula (I) is arranged between the electron transport layer and the cathode.

10. Compound having the general Formula (I)

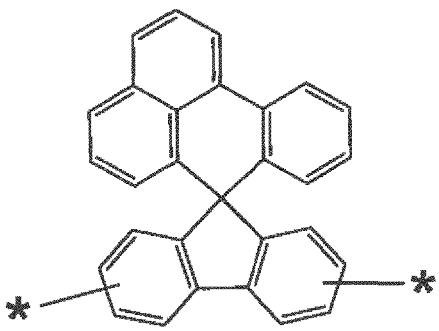

(I)

wherein

$$*-(A)_a-L$$

the compound of Formula (I) comprises one or more moieties $-(A)_a-L$ and the remaining positions are hydrogen or substituents independently selected from the group consisting of deuterium, fluorine, RF, $C_1$-$C_{20}$ linear alkyl, $C_3$-$C_{20}$ branched alkyl, $C_1$-$C_{12}$ linear fluorinated alkyl, CN, RCN, $C_6$-$C_{20}$ aryl, $C_2$-$C_{20}$ heteroaryl, $(P{=}O)R_2$; wherein each R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl;

A is selected from substituted or unsubstituted $C_6$-$C_{12}$ aryl or $C_2$-$C_{20}$ heteroaryl; wherein in case that A is substituted, the respective substituents are independently selected from the group consisting of deuterium, fluorine, $C_1$ - $C_{20}$ linear alkyl, $C_3$ - $C_{20}$ branched alkyl, linear fluorinated $C_1$ - $C_{12}$ alkyl, CN, $C_6$-$C_{20}$ aryl, and $C_2$ - $C_{20}$ heteroaryl;

L is selected from the group consisting of triazine, pyrimidine, benzoacridine, dibenzoacridine, pyridine, bipyridine, benzimidazole, phenanthroline, benzonitrile, phenanthridine, benzooxazole, benzothiazole, phenanthridine-one, naphto-benzofurane, di-naphtofurane benzo-naphto-thiophene, dinaphtothiophene, or a polar group selected from

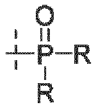 , 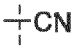 and 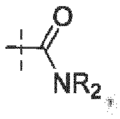,

wherein substituents, if present in the respective group L are independently selected from the group consisting of deuterium, fluorine, $C_1$-$C_{20}$ linear alkyl, $C_3$-$C_{20}$ branched alkyl, $C_3$-$C_{20}$ cyclic alkyl, $C_1$-$C_{20}$ linear alkoxy, $C_3$-$C_{20}$ branched alkoxy, $C_1$ - $C_{12}$ linear fluorinated alkyl, $C_1$-$C_{12}$ linear fluorinated alkoxy, $C_3$-$C_{12}$ branched fluorinated cyclic alkyl, $C_3$-$C_{12}$ fluorinated cyclic alkyl, $C_3$-$C_{12}$ fluorinated cyclic alkoxy, CN, RCN, $C_6$-$C_{20}$ aryl, $C_2$-$C_{20}$ heteroaryl, OR, SR, (C=O)R, (C=O)$NR_2$, $SiR_3$, (S=O)R, $(S=O)_2R$, $(P=O)R_2$; wherein each R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_2$-$C_{20}$ heteroaryl;

wherein in case that L is a N-containing heteroaryl group, the compound of the Formula (I) does only comprise one *-$(A)_a$-L moiety in which the $C_2$-$C_{42}$ heteroaryl is a N-containing heteroaryl group.

**11.** Organic semiconducting layer comprising the compound according to claim 10.

**12.** Display device comprising the organic electronic device according to any of the claims 1 to 9.

**13.** Lighting device comprising the organic electronic device according to any of the claims 1 to 9.

## Patentansprüche

**1.** Organische elektronische Vorrichtung, umfassend zwischen einer Anode und einer Kathode mindestens eine Schicht, die aus einer Elektroneninjektionsschicht, einer Elektronentransportschicht oder einer Elektronenerzeugungsschicht ausgewählt ist, wobei die Schicht mindestens eine Verbindung der folgenden Formel (I) umfasst:

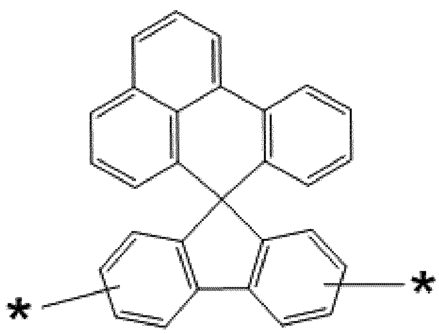

(I)

wobei

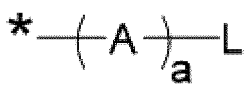

die Verbindung der Formel (I) eine oder mehrere Gruppierungen -$(A)_a$-L umfasst und die verbleibenden Positionen, die mit "*" markiert sind, Wasserstoff oder Substituenten sind, die unabhängig aus der Gruppe ausgewählt sind, bestehend aus Deuterium, Fluor, HF, linearem $C_1$-$C_{20}$-Alkyl, verzweigtem $C_3$-$C_{20}$-Alkyl, linearem fluorierten $C_1$-$C_{12}$-Alkyl, CN, RCN, $C_6$-$C_{20}$-Aryl, $C_2$-$C_{20}$-Heteroaryl, $(P=O)R_2$; wobei jedes R unabhängig aus linearem $C_1$-$C_{20}$-Alkyl, $C_1$-$C_{20}$-Alkoxy, $C_1$-$C_{20}$-Thioalkyl, verzweigtem $C_3$-$C_{20}$-Alkyl, cyclischem $C_3$-$C_{20}$-Alkyl, verzweigtem $C_3$-$C_{20}$-Alkoxy, cyclischem $C_3$-$C_{20}$-Alkoxy, verzweigtem $C_3$-$C_{20}$-Thioalkyl, cyclischem $C_3$-$C_{20}$-Thioalkyl, $C_6$-$C_{20}$-Aryl und C2-$C_{20}$-Heteroaryl ausgewählt ist;

A aus substituiertem oder unsubstituiertem $C_6$-$C_{24}$-Aryl oder $C_2$-$C_{20}$-Heteroaryl ausgewählt ist; wobei im Falle, dass A substituiert ist, die jeweiligen Substituenten unabhängig aus der Gruppe ausgewählt sind, die aus Deuterium, Fluor, linearem $C_1$-$C_{20}$-Alkyl, verzweigtem $C_3$-$C_{20}$-Alkyl, linearem fluoriertem $C_1$-$C_{12}$-Alkyl, CN,

$C_6$-$C_{20}$-Aryl und $C_2$-$C_{20}$-Heteroaryl besteht;
L aus der Gruppe ausgewählt ist, die aus Triazin, Pyrimidin, Benzoacridin, Dibenzoacridin, Pyridin, Bipyridin, Benzimidazol, Phenanthrolin, Benzonitril, Phenanthridin, Benzooxazol, Benzothiazol, Phenanthridin-on, Naphtobenzofuran, Di-naphtofuran, Benzonaphtothiophen, Dinaphtothiophen oder einer polaren Gruppe besteht, die aus

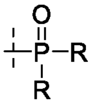 , 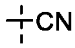 und 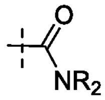

besteht, wobei Substituenten, falls in der jeweiligen Gruppe L vorhanden, unabhängig aus der Gruppe ausgewählt sind, die aus Deuterium, Fluor, linearem $C_1$-$C_{20}$-Alkyl, verzweigtem $C_3$-$C_{20}$-Alkyl, cyclischem $C_3$-$C_{20}$-Alkyl, linearem $C_1$-$C_{20}$-Alkoxy, verzweigtem $C_3$-$C_{20}$-Alkoxy, linearem fluoriertem $C_1$-$C_{12}$-Alkyl, linearem fluoriertem $C_1$-$C_{12}$-Alkoxy, verzweigtem fluoriertem $C_3$-$C_{12}$-Alkyl, fluoriertem cyclischem $C_3$-$C_{12}$-Alkyl, fluoriertem cyclischem $C_3$-$C_{12}$-Alkoxy, CN, RCN, $C_6$-$C_{20}$-Aryl, $C_2$-$C_{20}$-Heteroaryl, OR, SR, (C=O)R, (C=O)$NR_2$, $SiR_3$, (S=O)R, $(S=O)_2R$, $(P=O)R_2$ besteht; wobei jedes R unabhängig aus linearem $C_1$-$C_{20}$-Alkyl, $C_1$-$C_{20}$-Alkoxy, $C_1$-$C_{20}$-Thioalkyl, verzweigtem $C_3$-$C_{20}$-Alkyl, cyclischem $C_3$-$C_{20}$-Alkyl, verzweigtem $C_3$-$C_{20}$-Alkoxy, cyclischem $C_3$-$C_{20}$-Alkoxy, verzweigtem $C_3$-$C_{20}$-Thioalkyl, cyclischem $C_3$-$C_{20}$-Thioalkyl, cyclischem $C_3$-$C_{20}$-Thioalkyl, $C_6$-$C_{20}$-Aryl und $C_2$-$C_{20}$-Heteroaryl ausgewählt ist, und
"a" eine ganze Zahl von 0 bis 2 ist.

2. Organische elektronische Vorrichtung nach Anspruch 1, wobei A unsubstituiert ist.

3. Organische elektronische Vorrichtung nach Anspruch 1 oder 2, wobei im Falle, dass L substituiert ist, die jeweiligen Substituenten unabhängig aus der Gruppe ausgewählt sind, die aus Phenyl, Naphthyl, Pyridyl, Biphenylyl, Dibenzofuranyl, Dibenzothiophenyl und Carbazolyl besteht.

4. Organische elektronische Vorrichtung nach einem der vorhergehenden Ansprüche, wobei L aus der Gruppe ausgewählt ist, die aus Triazin, Pyrimidin, Benzoacridin und Dibenzoacridin besteht.

5. Organische elektronische Vorrichtung nach einem der vorhergehenden Ansprüche, wobei im Falle, dass L substituiert ist, die Substituenten unabhängig aus der Gruppe ausgewählt sind, die aus Folgenden besteht:

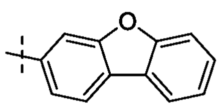 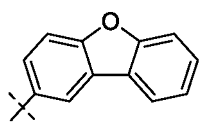 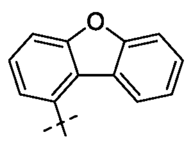 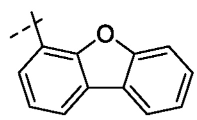

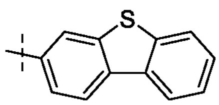 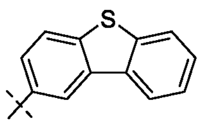 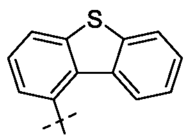 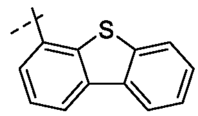

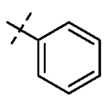 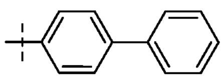 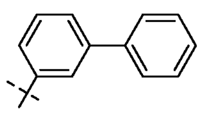 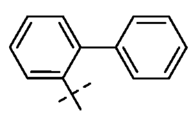

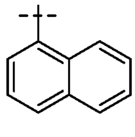 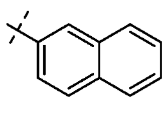 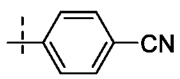 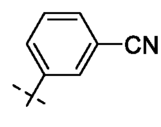

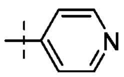 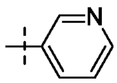 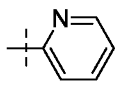 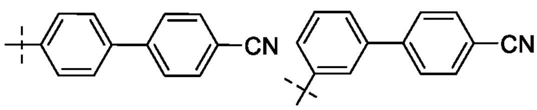

**6.** Organische elektronische Vorrichtung nach einem der vorhergehenden Ansprüche, wobei die Schicht, die die Verbindung der Formel (I) umfasst, aus mindestens einer Verbindung der Formel (I) besteht.

**7.** Organische elektronische Vorrichtung nach einem der vorhergehenden Ansprüche, wobei die Schicht, die die Verbindung der Formel (I) umfasst, ferner ein Metall, ein Metallsalz oder einen organischen Metallkomplex, vorzugsweise ein Alkalimetalladditiv oder ein Seltenerdmetalladditiv, stärker bevorzugt ein Seltenerdmetall oder ein Alkalimetallkomplex oder ein Alkalimetallsalz, stärker bevorzugt Yb oder LiQ oder Alkaliborat oder Alkaliphenolat, stärker bevorzugt LiQ umfasst.

**8.** Organische Halbleitendevorrichtung nach einem der vorhergehenden Ansprüche, wobei die organische elektronische Vorrichtung ferner eine Emissionsschicht umfasst und die Schicht, die die Verbindung der Formel (I) umfasst, zwischen der Emissionsschicht und der Kathode angeordnet ist.

**9.** Organische Halbleiterdevorrichtung nach einem der vorhergehenden Ansprüche, ferner umfassend eine Elektronentransportschicht, wobei die Schicht, die die Verbindung der Formel (I) umfasst, zwischen der Elektronentransportschicht und der Kathode angeordnet ist.

**10.** Verbindung mit der allgemeinen Formel (I)

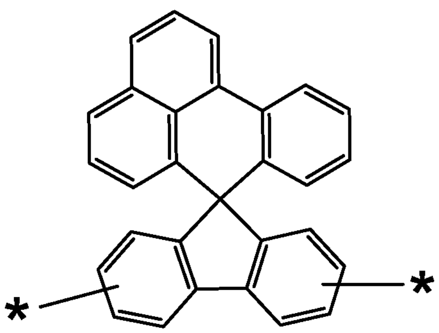

(I)

wobei

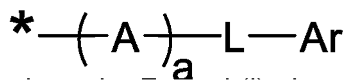

die Verbindung der Formel (I) eine oder mehrere Gruppierungen -$(A)_a$-L umfasst und die verbleibenden Positionen Wasserstoff oder Substituenten sind, die unabhängig aus der Gruppe ausgewählt sind, bestehend aus Deuterium, Fluor, HF, linearem $C_1$-$C_{20}$-Alkyl, verzweigtem $C_3$-$C_{20}$-Alkyl, linearem fluoriertem $C_1$-$C_{12}$-Alkyl, CN, RCN, $C_6$-$C_{20}$-Aryl, $C_2$-$C_{20}$-Heteroaryl, (P=O)R2; wobei jedes R unabhängig aus linearem C1-C20-Alkyl, C1-C20-Alkoxy, $C_1$-$C_{20}$-Thioalkyl, verzweigtem $C_3$-$C_{20}$-Alkyl, cyclischem $C_3$-$C_{20}$-Alkyl, verzweigtem $C_3$-$C_{20}$-Alkoxy, cyclischem $C_3$-$C_{20}$-Alkoxy, verzweigtem $C_3$-$C_{20}$-Thioalkyl, cyclischem $C_3$-$C_{20}$-Thioalkyl, $C_6$-$C_{20}$-Aryl und $C_2$-$C_{20}$-Heteroaryl ausgewählt ist;
A aus substituiertem oder unsubstituiertem $C_6$-$C_{12}$-Aryl oder $C_2$-$C_{20}$-Heteroaryl ausgewählt ist; wobei im Falle, dass A substituiert ist, die jeweiligen Substituenten unabhängig aus der Gruppe ausgewählt sind, die aus Deuterium, Fluor, linearem $C_1$-$C_{20}$-Alkyl, verzweigtem $C_3$-$C_{20}$-Alkyl, linearem fluoriertem $C_1$-$C_{12}$-Alkyl, CN, $C_6$-$C_{20}$-Aryl und $C_2$-$C_{20}$-Heteroaryl besteht;
L aus der Gruppe ausgewählt ist, die aus Triazin, Pyrimidin, Benzoacridin, Dibenzoacridin, Pyridin, Bipyridin, Benzimidazol, Phenanthrolin, Benzonitril, Phenanthridin, Benzooxazol, Benzothiazol, Phenanthridin-on, Naphtobenzofuran, Di-naphtofuran, Benzonaphtothiophen, Dinaphtothiophen oder einer polaren Gruppe besteht, die aus

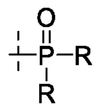 , 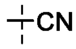 und 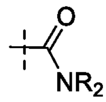

besteht, wobei Substituenten, falls in der jeweiligen Gruppe L vorhanden, unabhängig aus der Gruppe ausgewählt sind, die aus Deuterium, Fluor, linearem $C_1$-$C_{20}$-Alkyl, verzweigtem $C_3$-$C_{20}$-Alkyl, cyclischem $C_3$-$C_{20}$-Alkyl, linearem $C_3$-$C_{20}$-Alkoxy, verzweigtem $C_3$-$C_{20}$-Alkoxy, linearem fluoriertem $C_1$-$C_{12}$-Alkyl, linearem fluoriertem $C_1$-$C_{12}$-Alkoxy, verzweigtem fluoriertem $C_3$-$C_{12}$-Alkyl, fluoriertem cyclischem $C_3$-$C_{12}$-Alkyl, fluoriertem cyclischem $C_3$-$C_{12}$-Alkoxy, CN, RCN, $C_6$-$C_{20}$-Aryl, $C_2$-$C_{20}$-Heteroaryl, OR, SR, (C=O)R, (C=O)$NR_2$, $SiR_3$, (S=O)R, $(S=O)_2R$, $(P=O)R_2$ besteht; wobei jedes R unabhängig aus linearem $C_1$-$C_{20}$-Alkyl, linearem $C_1$-$C_{20}$-Alkoxy, $C_1$-$C_{20}$-Thioalkyl, verzweigtem $C_3$-$C_{20}$-Alkyl, cyclischem $C_3$-$C_{20}$-Alkyl, verzweigtem $C_3$-$C_{20}$-Alkoxy, cyclischem $C_3$-$C_{20}$-Alkoxy, verzweigtem $C_3$-$C_{20}$-Alkoxy, verzweigtem $C_3$-$C_{20}$-Thioalkyl, verzweigtem $C_3$-$C_{20}$-Thioalkyl, verzweigtem $C_3$-$C_{20}$-Alkoxy, verzweigtem $C_3$-$C_{20}$-Thioalkyl ausgewählt ist;
wobei im Falle, dass L eine N-enthaltende Heteroarylgruppe ist, die Verbindung der Formel (I) nur eine $*-(A)_a-L$-Gruppierung umfasst, in der das $C_2$-$C_{42}$-Heteroaryl eine N-enthaltende Heteroarylgruppe ist.

**11.** Organische Halbleiterdeschicht, umfassend die Verbindung nach Anspruch 10.

**12.** Anzeigevorrichtung, umfassend die organische elektronische Vorrichtung nach einem der Ansprüche 1 bis 9.

**13.** Beleuchtungsvorrichtung, umfassend die organische elektronische Vorrichtung nach einem der Ansprüche 1 bis 9.

## Revendications

**1.** Dispositif électronique organique comprenant, entre une anode et une cathode, au moins une couche choisie parmi une couche d'injection d'électrons, une couche de transport d'électrons ou une couche de génération d'électrons, la couche comprenant au moins un composé de la formule (I) suivante :

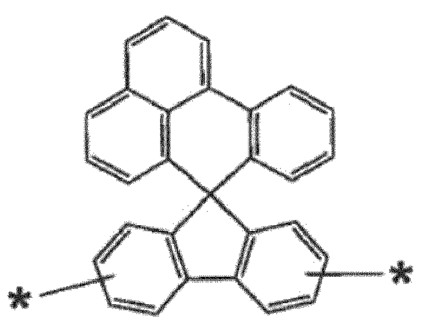

(I)

dans laquelle

$$*-(A)_a-L$$

le composé de formule (I) comprend une ou plusieurs fractions $-(A)_a$-L et les positions restantes marquées d'un « * » représentent l'hydrogène ou des substituants indépendamment choisis dans le groupe constitué par le deutérium, le fluor, RF, un alkyle linéaire en $C_1$-$C_{20}$, un alkyle ramifié en $C_3$-$C_{20}$, un alkyle fluoré linéaire en $C_1$-$C_{12}$, CN, RCN, un aryle en $C_6$-$C_{20}$, un hétéroaryle en $C_2$-$C_{20}$, $(P=O)R_2$ ; dans laquelle chaque R est indépendamment choisi parmi un alkyle linéaire en $C_1$-$C_{20}$, un alcoxy en $C_1$-$C_{20}$, un thioalkyle en $C_1$-$C_{20}$, un alkyle ramifié en $C_3$-$C_{20}$, un alkyle cyclique en $C_3$-$C_{20}$, un alcoxy ramifié en $C_3$-$C_{20}$, un alcoxy cyclique en $C_3$-$C_{20}$, un thioalkyle ramifié en $C_3$-$C_{20}$, un thioalkyle cyclique en $C_3$-$C_{20}$, un aryle en $C_6$-$C_{20}$, et un hétéroaryle en $C_2$-$C_{20}$ ; A est choisi parmi un aryle en $C_6$-$C_{24}$ ou un hétéroaryle en $C_2$-$C_{20}$ substitué ou non substitué ; dans laquelle dans le cas où A est substitué, des substituants respectifs sont indépendamment choisis dans le groupe constitué

par le deutérium, le fluor, un alkyle linéaire en $C_1$-$C_{20}$, un alkyle ramifié en $C_3$-$C_{20}$, un alkyle fluoré linéaire en $C_1$-$C_{12}$, CN, un aryle en $C_6$-$C_{20}$, et un hétéroaryle en $C_2$-$C_{20}$ ;
L est choisi dans le groupe constitué par la triazine, la pyrimidine, la benzoacridine, la dibenzoacridine, la pyridine, la bi-pyridine, le benzimidazole, la phénanthroline, le benzonitrile, la phénanthridine, le benzooxazole, le benzothiazole, la phénanthridine-one, le naphto-benzofurane, le di-naphtofurane, le benzo-naphto-thiophène, le dinaphtothiophène, ou un groupe polaire choisi parmi

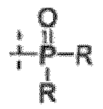 , 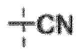 et 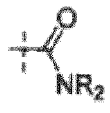 ,

dans laquelle les substituants, si présents dans le groupe L respectif sont indépendamment choisis dans le groupe constitué par le deutérium, le fluor, un alkyle linéaire en $C_1$-$C_{20}$, un alkyle ramifié en $C_3$-$C_{20}$, un alkyle cyclique en $C_3$-$C_{20}$, un alcoxy linéaire en $C_1$-$C_{20}$, un alcoxy ramifié en $C_3$-$C_{20}$, un alkyle fluoré linéaire en $C_1$-$C_{12}$, un alcoxy fluoré linéaire en $C_1$-$C_{12}$, un alkyle cyclique fluoré ramifié en $C_3$-$C_{12}$, un alkyle cyclique fluoré en $C_3$-$C_{12}$, un alcoxy cyclique fluoré en $C_3$-$C_{12}$, CN, RCN, un aryle en $C_6$-$C_{20}$, un hétéroaryle en $C_2$-$C_{20}$, OR, SR, (C=O)R, (C=O)$NR_2$, $SiR_3$, (S=O)R, $(S=O)_2R$, $(P=O)R_2$ ; dans laquelle chaque R est indépendamment choisi parmi un alkyle linéaire en $C_1$-$C_{20}$, un alcoxy en $C_1$-$C_{20}$, un thioalkyle en $C_1$-$C_{20}$, un alkyle ramifié en $C_3$-$C_{20}$, un alkyle cyclique en $C_3$-$C_{20}$, un alcoxy ramifié en $C_3$-$C_{20}$, un alcoxy cyclique en $C_3$-$C_{20}$, un thioalkyle ramifié en $C_3$-$C_{20}$, un thioalkyle cyclique en $C_3$-$C_{20}$, un aryle en $C_6$-$C_{20}$, et un hétéroaryle en $C_2$-$C_{20}$, et
« a » est un nombre entier compris entre 0 et 2.

**2.** Dispositif électronique organique selon la revendication 1, dans lequel A est non substitué.

**3.** Dispositif électronique organique selon la revendication 1 ou la revendication 2, dans lequel dans le cas où L est substitué, les substituants respectifs sont indépendamment choisis dans le groupe constitué par le phényle, le naphtyle, le pyridyle, le bi-phénylyle, le dibenzofuranyle, le dibenzothiophényl et le carbazolyle.

**4.** Dispositif électronique organique selon l'une quelconque des revendications précédentes, dans lequel L est choisi dans le groupe constitué par la triazine, la pyrimidine, la benzoacridine et la dibenzoacridine.

**5.** Dispositif électronique organique selon l'une quelconque des revendications précédentes, dans lequel dans le cas où L est substitué, les substituants respectifs sont indépendamment choisis dans le groupe constitué par

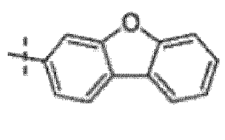 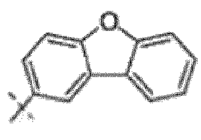 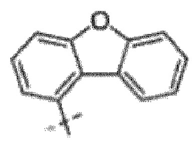 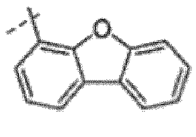

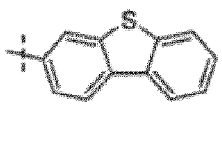 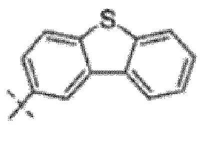 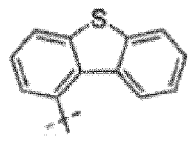 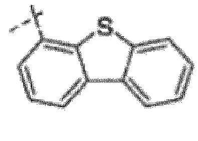

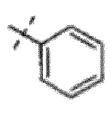 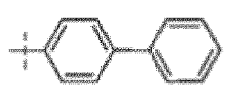 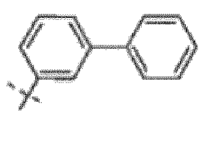 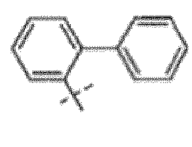

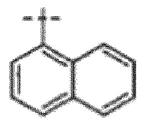 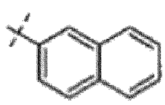 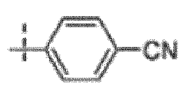 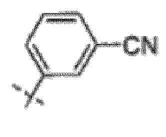

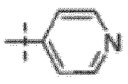 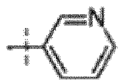 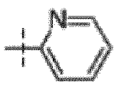 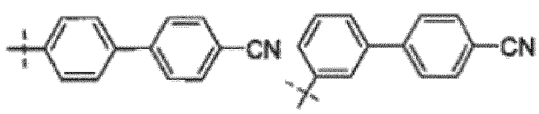 .

6. Dispositif électronique organique selon l'une quelconque des revendications précédentes, dans lequel la couche comprenant le composé de formule (I) est constituée d'au moins un composé de formule (I).

7. Dispositif électronique organique selon l'une quelconque des revendications précédentes, dans lequel la couche comprenant le composé de formule (I) comprend en outre un métal, un sel métallique ou un complexe organométallique, de préférence un additif de métal alcalin ou un additif de métal de terres rares, plus préférablement un métal de terres rares ou un complexe de métal alcalin ou un sel de métal alcalin, plus préférablement Yb ou LiQ ou un borate alcalin ou un phénolate alcalin, le plus préférablement LiQ.

8. Dispositif semi-conducteur organique selon l'une quelconque des revendications précédentes, dans lequel le dispositif électronique organique comprend en outre une couche d'émission et la couche comprenant le composé de formule (I) est agencé entre la couche d'émission et la cathode.

9. Dispositif semi-conducteur organique selon l'une quelconque des revendications précédentes, comprenant en outre une couche de transport d'électrons, dans lequel la couche comprenant le composé de formule (I) est agencé entre la couche de transport d'électrons et la cathode.

10. Composé ayant la formule générale (I)

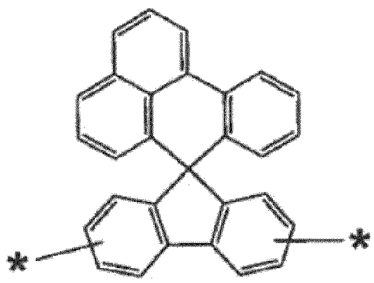

(I)

dans laquelle

$$*\!-\!(A)_a\!-\!L$$

le composé de formule (I) comprend une ou plusieurs fractions $-(A)_a$-L et les positions restantes représentent l'hydrogène ou des substituants indépendamment choisis dans le groupe constitué par le deutérium, le fluor, RF, un alkyle linéaire en $C_1$-$C_{20}$, un alkyle ramifié en $C_3$-$C_{20}$, un alkyle fluoré linéaire en $C_1$-$C_{12}$, CN, RCN, un aryle en $C_6$-$C_{20}$, un hétéroaryle en $C_2$-$C_{20}$, $(P{=}O)R_2$ ; dans laquelle chaque R est indépendamment choisi parmi un alkyle linéaire en $C_1$-$C_{20}$, un alcoxy en $C_1$-$C_{20}$, un thioalkyle en $C_1$-$C_{20}$, un alkyle ramifié en $C_3$-$C_{20}$, un alkyle cyclique en $C_3$-$C_{20}$, un alcoxy ramifié en $C_3$-$C_{20}$, un alcoxy cyclique en $C_3$-$C_{20}$, un thioalkyle ramifié en $C_3$-$C_{20}$, un thioalkyle cyclique en $C_3$-$C_{20}$, un aryle en $C_6$-$C_{20}$, et un hétéroaryle en $C_2$-$C_{20}$ ;

A est choisi parmi un aryle en $C_6$-$C_{12}$ ou un hétéroaryle en $C_2$-$C_{20}$ substitué ou non substitué ; dans laquelle dans le cas où A est substitué, les substituants respectifs sont indépendamment choisis dans le groupe constitué par le deutérium, le fluor, un alkyle linéaire en $C_1$-$C_{20}$, un alkyle ramifié en $C_3$-$C_{20}$, un alkyle fluoré linéaire en $C_1$-$C_{12}$, CN, un aryle en $C_6$-$C_{20}$, et un hétéroaryle en $C_2$-$C_{20}$ ;

L est choisi dans le groupe constitué par la triazine, la pyrimidine, la benzoacridine, la dibenzoacridine, la pyridine, la bi-pyridine, le benzimidazole, la phénanthroline, le benzonitrile, la phénanthridine, le benzooxazole, le benzothiazole, la phénanthridine-one, le naphto-benzofurane, le di-naphtofurane, le benzo-naphto-thiophène, le dinaphtothiophène, ou un groupe polaire choisi parmi

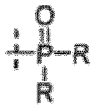 , 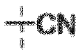 et 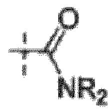,

dans laquelle les substituants, si présents dans le groupe L respectif sont indépendamment choisis dans le groupe constitué par le deutérium, le fluor, un alkyle linéaire en $C_1$-$C_{20}$, un alkyle ramifié en $C_3$-$C_{20}$, un alkyle cyclique en $C_3$-$C_{20}$, un alcoxy linéaire en $C_1$-$C_{20}$, un alcoxy ramifié en $C_3$-$C_{20}$, un alkyle fluoré linéaire en $C_1$-$C_{12}$, un alcoxy fluoré linéaire en $C_1$-$C_{12}$, un alkyle cyclique fluoré ramifié en $C_3$-$C_{12}$, un alkyle cyclique fluoré en $C_3$-$C_{12}$, un alcoxy cyclique fluoré en $C_3$-$C_{12}$, CN, RCN, un aryle en $C_6$-$C_{20}$, un hétéroaryle en $C_2$-$C_{20}$, OR, SR, (C=O)R, (C=O)$NR_2$, $SiR_3$, (S=O)R, $(S=O)_2R$, $(P=O)R_2$ ; dans laquelle chaque R est indépendamment choisi parmi un alkyle linéaire en $C_1$-$C_{20}$, un alcoxy en $C_1$-$C_{20}$, un thioalkyle en $C_1$-$C_{20}$, un alkyle ramifié en $C_3$-$C_{20}$, un alkyle cyclique en $C_3$-$C_{20}$, un alcoxy ramifié en $C_3$-$C_{20}$, un alcoxy cyclique en $C_3$-$C_{20}$, un thioalkyle ramifié en $C_3$-$C_{20}$, un thioalkyle cyclique en $C_3$-$C_{20}$, un aryle en $C_6$-$C_{20}$, et un hétéroaryle en $C_2$-$C_{20}$ ;
dans laquelle dans le cas où L est un groupe hétéroaryle contenant N, le composé de formule (I) ne comprend qu'une fraction *-$(A)_a$-L dans laquelle l'hétéroaryle en $C_2$-$C_{42}$ est un groupe hétéroaryle contenant N.

**11.** Couche semi-conductrice organique comprenant le composé selon la revendication 10.

**12.** Dispositif d'affichage comprenant le dispositif électronique organique selon l'une quelconque des revendications 1 à 9.

**13.** Dispositif d'éclairage comprenant le dispositif électronique organique selon l'une quelconque des revendications 1 à 9.

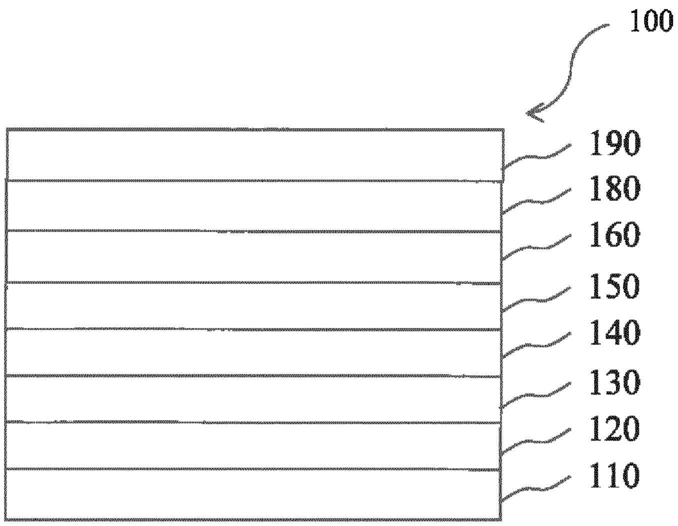
100
190
180
160
150
140
130
120
110

Fig.1

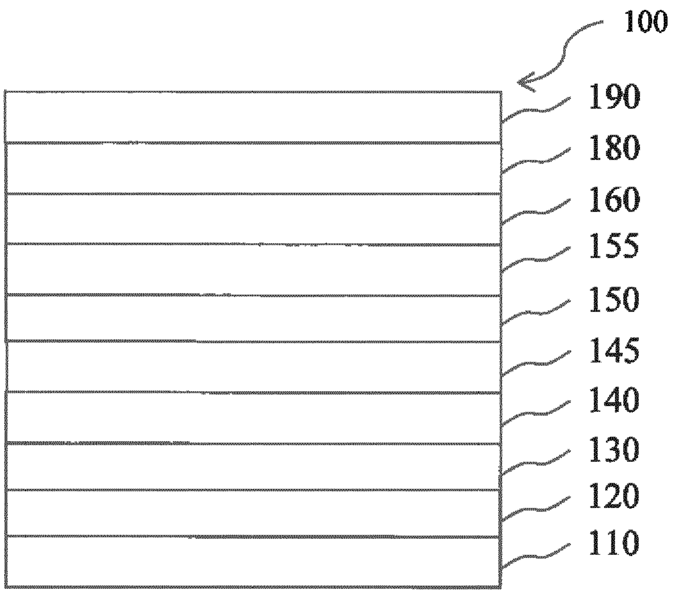
100
190
180
160
155
150
145
140
130
120
110

Fig.2

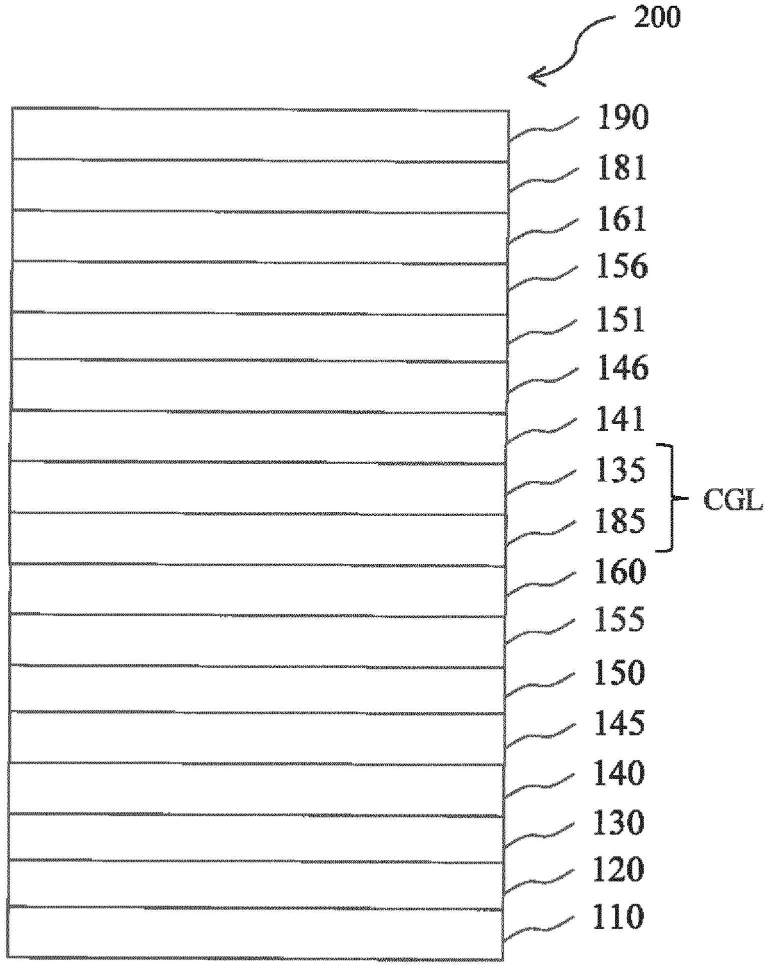
200
190
181
161
156
151
146
141
135
185
CGL
160
155
150
145
140
130
120
110

Fig.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2722908 A1
- EP 1970371 A1
- WO 2013079217 A1
- US 2016322581 A
- US 2008265216 A
- JP 2014096418 B
- WO 2016171358 A
- KR 101537500
- WO 2013079217 A


### Non-patent literature cited in the description


- **KIM J-Y et al.** *Dyes and Pigments,* 2012, vol. 94 (2), 304-313
- **YASUHIKO SHIROTA ; HIROSHI KAGEYAMA.** *Chem. Rev.,* 2007, vol. 107, 953-1010